# EUROPEAN PATENT SPECIFICATION

(11) **EP 3 303 604 B1**
(45) Date of publication and mention of the grant of the patent: **07.08.2019**
(21) Application number: 16734070.2
(22) Date of filing: 01.06.2016
(51) Int. Cl.: G01N 33/66, C12Q 1/00, A61B 5/145, A61L 2/08

(54) **PROTECTIVE AGENTS AGAINST E-BEAM IRRADIATION FOR PROTEINS IN OPTICAL SENSING CHEMISTRY**
SCHUTZMITTEL GEGEN E-BESTRAHLUNG FÜR PROTEINE IN DER OPTISCHEN SENSORCHEMIE
AGENTS DE PROTECTION DES PROTÉINES CONTRE UNE EXPOSITION À UN FAISCEAU ÉLECTRONIQUE EN CHIMIE PAR DÉTECTION OPTIQUE

(30) Priority: 02.06.2015 US 201514728442
(43) Date of publication of application: 11.04.2018
(73) Proprietor: Medtronic MiniMed, Inc., Northridge, CA 91325-1219 (US)
(72) Inventor: DANG, Tri T., Winnetka, CA 91306 (US); AROYAN, Sarkis, Northridge, CA 91326 (US); KRISTENSEN, Jesper Svenning, 2830 Virum (DK)
(74) Representative: Ruschke, Hans Edvard
(86) International application number: PCT/US2016/035315
(87) International publication number: WO 2016/196662

(56) References cited:
- EP-A1- 1 430 831
- WO-A1-2013/074668
- WO-A2-2007/022485
- WO-A2-2011/115809
- US-A1- 2003 161 753
- VON WOEDTKE TH ET AL: "Sterilization of enzyme glucose sensors: problems and concepts", BIOSENSORS AND BIOELECTRONICS, ELSEVIER BV, NL, vol. 17, no. 5, 1 May 2002 (2002-05-01), pages 373-382, XP002572822, ISSN: 0956-5663

## Description

### BACKGROUND OF THE INVENTION

### 1. Field of the Invention

This invention relates to medical devices useful in *in vivo* environments, in particular, methods and materials used to protect the sterilization of such devices prior to their implantation *in vivo.*

### 2. Description of Related Art

Medical personnel and patients commonly utilize a wide variety of pre-sterilized medical products, such as glucose sensors that are used by diabetic patients. In this context, a number of different sterilization processes are used with various medical products in order to kill microorganisms that may be present. Most sterilization processes require the sterilizing agent to systemically permeate the article being sterilized. These methods can include heat sterilization, where the object to be sterilized is subjected to heat and pressure, such as in an autoclave. The heat and pressure penetrates though the object being sterilized and after a sufficient time will kill any harmful microorganisms. Gases such as hydrogen peroxide or ethylene oxide may also be used to sterilize objects. Sterilization methods also include those that use ionizing radiation, such as gamma-rays, x-rays, or energetic electrons to kill microorganisms.

Radiation has a number of advantages over other sterilization processes 'including a high penetrating ability, relatively low chemical reactivity, and instantaneous effects without the need to control temperature, pressure, vacuum, or humidity. Consequently, the sterilization of medical devices by exposure to radiation is a common practice. Medical devices composed in whole or in part of polymers are typically sterilized by various kinds of radiation, including, but not limited to, electron beam (e-beam), gamma ray, ultraviolet, infra-red, ion beam, and x-ray sterilization..

Electron-beam and gamma ray sterilization processes provide forms of radiation commonly used to kill microbial organisms on medical devices. However, when used to kill microorganisms, such radiation can alter the structure of functional macromolecules present in medical products including polymers such as proteins. High-energy radiation tends to produce ionization and excitation in polymer molecules, as well as free radicals. These energy-rich species can react with macromolecules present in medical products and undergo dissociation, abstraction, chain scission and cross-linking.

The deterioration of the performance of polymeric materials and other macromolecules in medical devices due to radiation sterilization has been associated with free radical formation during radiation exposure. Electron-beam and gamma ray radiation can therefore be problematic when used to sterilize medical devices that include components which are radiation sensitive. This complicates the sterilization process and limits the range of designs or materials available for medical devices. Consequently, methods and formulations that can protect medical device materials from damage that can occur as a result of exposure to high-energy radiation are desirable.

WO 2013/074668 discloses a method for inhibiting damage from radiation sterilization to a saccharide sensor comprising a saccharide binding polypeptide. An aqueous radioprotectant formulation comprising a saccharide is used.

WO 2011/115809 discloses a radiation insensitive analyte sensor having at least one radiation stabilizing agent. Radiation stabilizing agents include hindered light amine stabilizers, antioxidants, vitamins and combinations thereof.

WO 2007/022485 discloses methods for sterilizing a biosensor. Irradiation sterilization is performed in an inert gas or other low oxygen environment achieved such as by vacuum packaging or packaging in the presence of an oxygen scavenger such as powdered iron oxide.

US 2003/0161753 disclose a methods for sterilizing a biological material that is sensitive to radiation including by adding a stabilizer. Suitable stabilizers include antioxidants and free radical scavengers.

### SUMMARY OF THE INVENTION

The invention is as defined in claim 1. Optional features are as set out in the dependent claims.

As noted above, the sterilization of medical.devices by exposure to radiation is a common practice. Unfortunately, radiation sterilization can compromise the function of certain components of some medical devices. In this context, embodiments of the invention provide methods and materials that can be used to protect medical devices from the unwanted effects of radiation sterilization.

The electron beam (e-beam) sterilization of sensors used in optical glucose sensing can greatly reduce the glucose sensitivity of such sensors. The disclosure provided herein describes formulations useful in such sterilization processes. These formulations include reactive oxygen species (ROS), such as nitrates, sulphates, and phosphates and other electron accepting compounds. These agents are included in the formulations in a manner that allows such compounds to act as radical oxidative scavengers that can protect proteins (e.g. glucose oxidase) and other radiation sensitive sensor components (e.g. fluorescent sensing moieties) by absorbing the free electron energy and mitigating the damage caused by e-beam irradiation processes. As discussed below, glucose sensors irradiated in ROS formulations retain higher levels of glucose sensitivity than control formulations lacking such excipients.

The invention disclosed herein has a number of embodiments. Typical embodiments of the invention comprise methods for inhibiting damage to a saccharide sensor that can result from a radiation sterilization process (e.g. electron beam irradiation) by combining the saccharide sensor with an aqueous radioprotectant formulation during the sterilization process. In common embodiments of the invention, the saccharide sensor comprises a saccharide binding polypeptide having a carbohydrate recognition domain and the aqueous radioprotectant formulation comprises a saccharide selected for its ability to bind the saccharide binding polypeptide. The aqueous radioprotectant formulation further comprises a reactive oxygen species. In described arrangements, the reactive oxygen species is a nitrate, sulphate, phosphate or benzoyl peroxide. In specific embodiments, the reactive oxygen species is sodium nitrate. In other embodiments of the invention, the radioprotectant formulation further comprises mannitol. In illustrative embodiments of the invention, the sensor is a glucose sensor and the saccharide binding polypeptide comprises mannan binding lectin, concanavalin A, glucose-galactose binding protein, or glucose oxidase. In certain methods of the invention, the sterilization process is performed under conditions selected so that the saccharide binds the saccharide binding polypeptide and the reactive oxygen species absorbs free electron energy generated by the radiation sterilization process, thereby inhibiting damage to the saccharide sensor.

As discussed below, a number of compounds are useful in the radioprotectant formulations disclosed herein. In one or more described arrangements, the radioprotectant formulation comprises a reactive oxygen species (ROS) such as nitrate, sodium nitrate, benzoyl peroxide, glutathione, superoxide dismutase, hydroxyethyl acrylate, and PEG. In certain arrangements, the aqueous radioprotectant formulation comprises a saccharide such as glucose, mannose, fructose, melizitose, N-acetyl-D-glucosamine, sucrose or trehalose. In some arrangements, the aqueous radioprotectant formulation comprises an antioxidant selected from the group consisting of ascorbate, urate, nitrite, vitamin E, α-tocopherol or nicotinate methylester. In certain arrangements of the invention, the aqueous; radioprotectant formulation comprises a buffering agent, for example, one selected from the group consisting of citrate, tris(hydroxymethyl)aminomethane (TRIS) and tartrate. In various embodiments of the invention the radioprotectant formulations can comprise additional agents such as surfactants, amino acids, pharmaceutically acceptable salts and the like.

Related described arrangements include compositions of matter comprising a medical device combined with an aqueous radioprotective formulation.

One arrangement is a composition of matter comprising a saccharide sensor that includes a saccharide binding polypeptide having a carbohydrate recognition domain. In typical compositions, a saccharide sensor is combined with an aqueous radioprotectant formulation comprising a saccharide, wherein the saccharide binds to the saccharide binding polypeptide, and a reactive oxygen species. Optionally in such compositions, the saccharide sensor includes a fluorophore and is combined with a fluorophore quenching compound in the aqueous radioprotective formulation.

A number of compounds can be combined with the saccharide sensors disclosed herein to form radioprotectant compositions. In one or more described arrangements, the composition comprises a reactive oxygen species (ROS) such as nitrate, sodium nitrate, benzoyl peroxide, glutathione, superoxide dismutase, hydroxyethyl acrylate, and PEG. In typical arrangements of : the invention, the composition comprises a saccharide selected from the group consisting of glucose, mannose, fructose, melizitose, N-acetyl-D-glucosamine, sucrose or trehalose. In certain arrangements, the composition comprises a fluorophore quenching compound, for example, acetaminophen. In some
arrangements, the composition comprises an antioxidant compound selected from the group consisting of ascorbate, urate, nitrite, vitamin E, α-tocopherol or nicotinate methylester. In some arrangements, the composition comprises a surfactant, for example a polysorbate such as Tween 80. In certain
arrangements, the composition comprises a buffering agent such as citrate, tris(hydroxymethyl)aminomethane (TRIS) or tartrate.

### BRIEF DESCRIPTION OF THE FIGURES

**Figure 1A** shows a sensor design comprising a tubular capsule that is implanted under the skin and provides an optical sensor in response to analyte (glucose). **Figure 1B** shows a view of this capsule. **Figure 1C** shows the relative size of this capsule. **Figure ID** shows a diagram of an alternative sensor design, one comprising an amperometric analyte sensor formed from a plurality of planar layered elements.
**Figure 2** shows a bar graph of data presenting dose response (DR) retention as a function of e-beam radiation dose for non-formulated sensors (control sensors not combined with any radioprotectant compositions), triple dose and formulated sensors at 15 kGy. The triple dose is 3×5 kGy. The sensors tested were radiated wet in a solution comprising 50 mM Tris-buffer saline. The arrow symbolizes 80% or more of DR can be retained after the formulated sensors are exposed to 15 kGy.
**Figure 3** shows a plot of phase and intensity data obtained from sensors after exposure to 15 kGy of radiation. The dose response is 1.7 after radiation compared to 2.1 before radiation (*i.e.* a retention of 81%).
**Figure 4** shows a graph of data on DR retained for irradiated sensors as a function of ascorbate concentration used for formulation. Utilizing too low or too high concentrations of ascorbate both yielded low retained DR, whereas the 20 mM to 100 mM concentration range yielded good protection.
**Figure 5** shows a graph of data on DR retained for irradiated sensors as a function of acetaminophen (also known as paracetamol, abbreviated PAM) concentration used for formulation. It is seen that using low concentrations of acetaminophen yields low retained DR whereas the use of concentrations above 10 mM yields good protection. Further it is shown that adding ascorbate to the excipients in most cases provides better protective effects.
**Figure 6** shows a graph of data on DR retained for irradiated sensors as a function of acetaminophen concentration used for formulation.
**Figure 7** shows a graph of data of DR retained for irradiated sensors as a function of acetaminophen concentration used for formulation. All sensors have contained 100 mM sucrose and variation of additions of ascorbate and mannose are also shown.
**Figure 8** shows a graph of data of DR retained for irradiated sensors as a function of ascorbate concentration used for formulation. All sensors have contained 500 mM sucrose and variation of additions of acetaminophen (PAM) and mannose are also shown.
**Figure 9** shows a bar graph of data presenting the absolute DR for both radiated and non-radiated sensor as a function of formulating the sensors with acetaminophen and ascorbic acid/ascorbate.
**Figure 10** shows a bar graph of data presenting the absolute DR for both radiated and non-radiated sensor as a function of formulating the sensors with acetaminophen, ascorbic acid, mannose and 500 mM sucrose. The overall result is illustrated in Figure 11.
**Figure 11** shows a graph of data showing sensor response after using Tris/Citrate saline buffer and excipients. Sensors show good retention of DR.
**Figure 12** shows a graph of data presenting a direct comparison of e-beamed and non e-beamed sensors.
**Figure 13** shows a graph of data obtained from a native sensor tested after storage in PBS pH=5.5. The sensor itself has no problem with the PBS buffer.
**Figure 14** shows a graph of data obtained from a sensor with excipients added (500 mM sucrose, 20 mM acetaminophen and 50 mM ascorbate) in PBS buffer during e-beam irradiation.
**Figure 15** shows a graph of data obtained from a sensor with excipients added (500 mM sucrose, 20 mM acetaminophen and 50 mM ascorbate) in PBS buffer.
**Figure 16** shows a bar graph of data on retained DR for using different buffer concentrations.
**Figure 17** shows a graph of data resulting from sensors using citrate only during e-beam irradiation.
**Figure 18** shows a graph of data resulting from sensors using citrate and excipients during e-beam irradiation.
**Figure 19** shows a graph of relative sensor performance with various excipients. The first 5 sensor groups contain 50 mM sucrose and 2 M bicarbonate in addition to other excipient mentioned. Group 6 "100mM NaNO3 with 320 mM man" comprises 50mM sucrose, 320mM mannitol, and 100mM sodium nitrate.
**Figure 20** shows a graph of the change of 50 mM sucrose, 320 mM mannitol, and 100 mM NaNO3 formulation ratios over time.
**Figure 21** shows a graph of sensor response retention after e-beam irradiation. All the groups contain 50mM sucrose. All the groups also contain 2 M bicarbonate except for the group with mannitol. Group 1 "0 mM NaNO3" further comprises 0 mM sodium nitrate, 25mM sodium ascorbate, and 10mM acetaminophen. Group 2 "100 mM NaNO3" further comprises 100 mM sodium nitrate. Group 3 "500 mM NaNO3" further comprises 500 mM sodium nitrate. Group 4 "1 M NaNO3" further comprises 1 M sodium nitrate. Group 5 "100 mM NaNO3 with 320 mM man" comprises 50mM sucrose, 320mM mannitol, and 100mM sodium nitrate.
**Figure 22** shows the chemical structures of various protective excipients (i.e. sodium nitrate, reactive oxygen species, mannitol, and benzoyl peroxide) described herein.
**Figure 23** shows a graph of sensor dose response of various formulations. Replacing bicarbonate with mannitol increased response by about 8%. Group 1 "Nominal, Control" comprises 50mM sucrose, 2M bicarbonate, 25mM sodium ascorbate, and 10mM acetaminophen. Group 2 "Bicarbonate and NaNO3 Repeat" and Group 3 "Bicarbonate and NaNO3 - No Dialysis" both comprise 50mM sucrose, 2M bicarbonate, and 100mM sodium nitrate. Group 4 "Mannitol and NaNO3" comprises 50mM sucrose, 320mM mannitol, and 100mM sodium nitrate.
**Figure 24** shows a graph of the change of 50 mM sucrose, 320 mM mannitol, and 100 mM NaNO3 formulation ratios over time.
**Figure 25** shows a graph of the change of 50 mM trehalose, 320 mM mannitol, and 100 µM benzoyl peroxide formulation ratios over time.
**Figure 26** shows a graph of sensor response retention with various excipients.

### DETAILED DESCRIPTION OF THE INVENTION

In the description of the preferred embodiment, reference is made to the accompanying drawings which form a part hereof, and in which is shown by way of illustration a specific embodiment in which the invention may be practiced. It is to be understood that other embodiments may be utilized and structural changes may be made without departing from the scope of the present invention.

Unless otherwise defined, all terms of art, notations and other scientific terms or terminology used herein are intended to have the meanings commonly understood by those of skill in the art to which this invention pertains. In some cases, terms with commonly understood meanings are defined herein for clarity and/or for ready reference, and the inclusion of such definitions herein should not necessarily be construed to represent a substantial difference over what is generally understood in the art. Many of the techniques and procedures described or referenced herein are well understood and commonly employed using conventional methodology by those skilled in the art. As appropriate, procedures involving the use of commercially available kits and reagents are generally carried out in accordance with manufacturer defined protocols and/or parameters unless otherwise noted.

The term "sensor" for example in "analyte sensor," is used in its ordinary sense, including, without limitation, means used to detect a compound such as an analyte. A "sensor system" includes, for example, elements, structures and architectures (e.g. specific 3-dimensional constellations of elements) designed to facilitate sensor use and function. Sensor systems can include, for example, compositions such as those having selected material properties, as well as electronic components such as elements and devices used in signal detection (e.g. optical detectors, current detectors, monitors, processors and the like). The term "sensing complex" as used herein refers to the elements of a sensor that interact with and generate a signal indicative of, a particular analyte (e.g. glucose and the like). The term "matrix" is used herein according to its art-accepted meaning of something within or from which something else is found, develops, and/or takes form.

While typical embodiments of the invention pertain to glucose sensors used in the management of diabetes, the systems, methods and materials disclosed herein can be adapted for use with a wide variety of medical devices known in the art. Furthermore, though many embodiments of the invention are described as relating to electron beam irradiation, various embodiments of the invention may also be applied to other forms of radiation sterilization processes, such as gamma ray sterilization.

In the management of diabetes, the regular measurement of glucose in the blood is essential in order to ensure correct insulin dosing. Furthermore, it has been demonstrated that in the long term care of a diabetic patient, better control of the blood glucose levels can delay, if not prevent, the onset of retinopathy, circulatory problems and other degenerative diseases often associated with diabetes. Thus, there is a need for reliable and accurate self-monitoring of blood glucose levels by diabetic patients. Typically, blood glucose is monitored by diabetic patients with the use of commercially available colorimetric test strips or electrochemical biosensors (e.g. enzyme electrodes), both of which require the regular use of a lancet-type instrument to withdraw a suitable amount of blood each time a measurement is made. On average, the majority of diabetic patients would use such instruments to take a measurement of blood glucose twice a day. However, the U.S. National Institute of Health has recommended that blood glucose testing should be carried out at least four times a day, a recommendation that has been endorsed by the American Diabetes Association. This increase in the frequency of blood glucose testing imposes a considerable burden on the diabetic patient, both in financial terms and in terms of pain and discomfort, particularly in the long-term diabetic who has to make regular use of a lancet to draw blood from the fingertips. Thus, there is clearly a need for a better long-term glucose monitoring system that does not involve drawing blood from the patient.

There have been a number of proposals for glucose measurement techniques that do not require blood to be withdrawn from the patient. One method for assaying glucose via competitive binding uses a proximity-based signal generating/modulating moiety pair which is typically an energy transfer donor acceptor pair (comprising an energy donor moiety and an energy acceptor moiety). The energy donor moiety is photoluminescent (usually fluorescent). In such methods, an energy transfer donor-acceptor pair is brought into contact with the sample (such as subcutaneous fluid) to be analyzed. The sample is then illuminated and the resultant emission detected. Either the energy donor moiety or the energy acceptor moiety of the donor-acceptor pair is bound to a receptor carrier (for example a carbohydrate binding molecule), while the other part of the donor acceptor pair (bound to a ligand carrier, for example a carbohydrate analogue) and any analyte (for example carbohydrate) present compete for binding sites on the receptor carrier. Energy transfer occurs between the donors and the acceptors when they are brought together. An example of donor-acceptor energy transfer is fluorescence resonance energy transfer (Förster resonance energy transfer, FRET), which is non-radiative transfer of the excited-state energy from the initially excited donor (D) to an acceptor (A). Energy transfer produces a detectable lifetime change (reduction) of the fluorescence of the energy donor moiety. Also, a proportion of the fluorescent signal emitted by the energy donor moiety is quenched. The lifetime change is reduced or even eliminated by the competitive binding of the analyte. Thus, by measuring the apparent luminescence lifetime, for example, by phase-modulation fluorometry or time resolved fluorometry (see Lakowicz, Principles of Fluorescence Spectroscopy, Plenum Press, 1983, Chapter 3), the amount of analyte in the sample can be determined. The intensity decay time and phase angles of the donor are expected to increase with increasing analyte concentration. An important characteristic of FRET is that it occurs over distances comparable to the dimensions of biological macromolecules. The distance at which FRET is 50% efficient, called the Förster distance, is typically in the range of 20-60 Å. Förster distances ranging from 20 to 90 Å are convenient for competitive binding studies. See, e.g. US Patent No. 6232120 and U.S. Patent Application Publication Nos. 20080188723, 20090221891, 20090187084 and 20090131773.

WO 91/09312 describes a subcutaneous method and device that employs an affinity assay based on glucose (incorporating an energy transfer donor acceptor pair) that is interrogated remotely by optical means. WO97/19188, WO 00/02048, WO 03/006992 and WO 02/30275 each describe glucose sensing by energy transfer, which produce an optical signal that can be read remotely. The systems discussed above rely on the plant lectin Concanavalin A (Con A) as the carbohydrate binding molecule. WO 06/061207 proposes that animal lectins such as mannose binding lectin (MBL) could be used instead. Previously disclosed carbohydrate analogues (e.g. those of US Patent No. 6,232,130) have comprised globular proteins to which carbohydrate and energy donor or energy acceptor moieties are conjugated. Carbohydrate polymers (e.g. optionally derivatized dextran and mannan) have also been used as carbohydrate analogues. In WO 06/061207 the use of periodate cleavage to allow binding of dextran to MBL at physiological calcium concentrations is disclosed. The assay components in such systems are typically retained by a retaining material. This may for example be a shell of biodegradable polymeric material, as described in WO 2005/110207.

Before implantable medical devices such as glucose sensors are introduced into the body, they must be sterilized. However, the materials of such devices, for example the assay components in sensors, can be sensitive to damage during sterilization. Heat sterilization causes denaturation of protein (lectin and/or carbohydrate analogue). Gas sterilization is difficult to use for wet devices such as the sensor. In view of this, the sterilization of medical devices by exposure to radiation is a common practice. Types of radiation which may be used in sterilization include gamma radiation and electron beam radiation. Electron beam radiation is easier to control than gamma radiation. However, electron beam radiation can lead to loss of protein activity and bleaching of dyes (e.g. a donor fluorophore and/or a acceptor dye). These effects can lead to loss of sensor activity.

Embodiments of the invention provide methods and materials that can be used to protect medical devices such as implantable glucose sensors from unwanted effects of radiation sterilization. The invention disclosed herein has a number of embodiments. Typical embodiments of the invention comprise methods for inhibiting damage to a medical device (e.g. a saccharide sensor) that can result from a radiation sterilization process by combining the medical device with an aqueous radioprotectant formulation during the sterilization process. In the context of embodiments of the invention as disclosed herein, because electron beam and gamma irradiation are fundamentally the same process, the protection provided by the methods and materials of the invention will be the same for these forms of irradiation. Gamma rays release secondary electrons from the materials around the item and hence create a cascade of electrons much like the e-beam. For this reason, gamma irradiation is suitable for sensors comprising one or more metal elements because metal is a good provider of secondary electrons. In some embodiments of the invention, the radiation sterilization process comprises electron beam irradiation. In some embodiments of the invention, the radiation sterilization process comprises gamma ray irradiation.

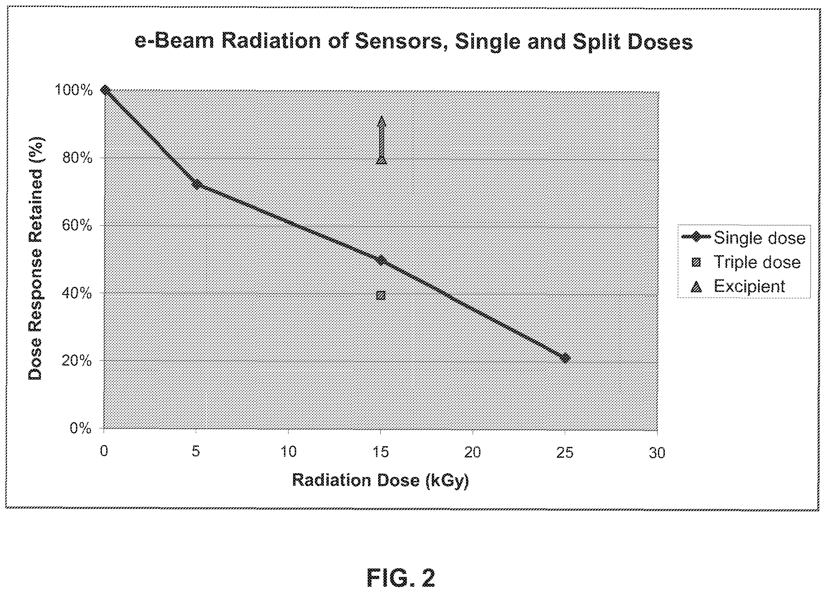

While the medical devices can be exposed to radiation supplied in multiple doses (e.g. 3×5 kGy for a total dose of 15 kGy), in typical embodiments of the instant invention, radiation is supplied in a single dose (e.g. 1 X 15 kGy for a total dose of 15 kGy). As disclosed herein (see, e.g. Figure 2), supplying a sterilizing amount radiation in a single dose gives better radiation protection than supplying the same amount of radiation in multiple doses (dividing the radiation into a triple dose resulted in sensors having worse signal retention). Optionally, the total dose of radiation is not more than 35 kGy, and typically is in the range of. 10-20 kGy). In certain embodiments the total dose is 15 kGy ± 2 kGy. Gy (J/kg) is the SI unit of dose i.e. the amount of energy absorbed per unit mass. Following radiation exposure, sensor function parameters can be evaluated such as the sensor Dose Response (DR relative to 0 kGy DR) as well as the absolute DR (measured in degrees phase shift from 40 mg/dL glucose to 400 mg/dL glucose). In certain embodiments of the invention, an aqueous radiation protecting formulation functions to protect a glucose sensor from radiation damage so that the glucose sensor retains at least 50, 60 or 70% of its dose response (DR) to glucose following irradiation of the sensor (as compared to the DR of a control sensor that received no irradiation).

In some embodiments of the invention, the saccharide sensor comprises a boronic acid derivative such as those disclosed in U.S. Patent Nos. 5,777,060, 6,002,954 and 6,766,183, the contents of which are incorporated herein by reference. In other embodiments of the invention, the saccharide sensor comprises a saccharide binding polypeptide. In certain embodiments of the invention the saccharide sensor comprises a lectin. Optionally the lectin is a C-type (calcium dependent) lectin. In some embodiments, the lectin is a vertebrate lectin, for example a mammalian lectin such as a human or humanized lectin. However, it may alternatively be a plant lectin, a bird lectin, a fish lectin or an invertebrate lectin such as an insect lectin. In certain embodiments, the lectin is in multimeric form. Multimeric lectins may be derived from the human or animal body. Alternatively, the lectin may be in monomeric form. Monomeric lectins may be formed by recombinant methods or by disrupting the binding between sub-units in a natural multimeric lectin derived from the human or animal body. Examples of this are described in U.S. Pat. No. 6,232,130. Saccharide sensors useful in embodiments of the invention are also disclosed in U.S. Patent Publication No. 2008/0188723.

In certain embodiments of the invention, the saccharide sensing element in a saccharide sensor comprises a lectin. Optionally, the lectin is mannose binding lectin, conglutinin or collectin-43 (e.g. bovine CL-43) (all serum collecting) or a pulmonary surfactant protein (lung collectins). Mannose binding lectin (also called mannan binding lectin or mannan binding protein, MBL, MBP), for example human MBL, has proved particularly interesting. MBL is a collagen-like defense molecule which comprises several (typically 3 to 4 (MALDI-MS), though distributions of 1 to 6 are likely to occur (SDS-PAGE)) sub-units in a "bouquet" arrangement, each composed of three identical polypeptides. Each sub-unit has a molecular weight of around 75 kDa, and can be optionally complexed with one or more MBL associated serine proteases (MASPs). Each polypeptide contains a CRD. Thus, each sub-unit presents three carbohydrate binding sites. Trimeric MBL and tetrameric MBL (which are the major forms present in human serum, Teillet et al., Journal of Immunology, 2005, page 2870-2877) present nine and twelve carbohydrate binding sites respectively. In typical embodiments of the invention, the lectin comprises polypeptides of *Homo sapiens* mannose-binding protein C precursor (NCBI Reference Sequence: NP_000233.1). Serum MBL is made of 3-4 subunits of 3 polypeptides each. The sequence of NCBI Reference Sequence: NP_000233.1 is between 27 kDa and 30 kDa giving the entire MBL protein a Mw typically of 270 kDa to 300 kDa.

Alternatively, the lectin may be a pulmonary surfactant protein selected from SP-A and SP-D. These proteins are similar to MBL. They are water-soluble collecting which act as calcium dependent carbohydrate binding proteins in innate host-defense functions. SP-D also binds lipids. SP-A has a "bouquet" structure similar to that of MBL (Kilpatrick D C (2000) Handbook of Animal Lectins, p. 37). SP-D has a tetrameric "X" structure with CRDs at each end of the "X". Other suitable animal lectins are known in the art such as PC-lectin CTL-1, Keratinocyte membrane lectins, CD94, P35 (synonym: human L-ficolin, a group of lectins), ERGIC-53 (synonym: MR60), HIP/PAP, CLECSF8, DCL (group of lectins), and the GLUT family proteins, especially GLUT1, GLUT4 and GLUT11. Further suitable animal lectins are set out in Appendices A, B and C of "Handbook of Animal Lectins: Properties and Biomedical Applications", David C. Kilpatrick, Wiley 2000.

In common embodiments of the invention, the saccharide sensor comprises a saccharide binding polypeptide having a carbohydrate recognition domain and the aqueous radioprotectant formulation comprises a saccharide selected for its ability to bind the saccharide binding polypeptide. In certain embodiments of the invention, the saccharide sensor comprises one or more fluorophores (e.g. a donor and/or a reference fluorophore); and the aqueous radioprotectant formulation comprises a fluorophore quenching compound selected for its ability to quench the fluorophore(s). Optionally, the sensor comprises at least one of protein/polypeptide, at least one energy donor, and/or at least one energy acceptor and this sensor is combined with at least one protective substance. In some embodiments the sensor comprises a protein, a fluorescent dye, dextran and a polymeric material. In illustrative embodiments of the invention, the sensor is a glucose sensor and the saccharide binding polypeptide comprises a mannan binding lectin, a concanavalin A, a glucose oxidase, or a glucose-galactose binding protein (see, e.g. U.S. Pat. No. 6,232,130; U.S. Patent Publication No. 2008/0188723; Jensen et al., Langmuir. 2012 Jul 31;28(30):11106-14. Epub 2012; Paek et al., Biosens Bioelectron. 2012 and Judge et al., Diabetes Technol Ther. 2011 Mar;13(3):309-17, 2011,).

As discussed below, a number of compounds are useful in the radioprotectant formulations disclosed herein. In certain embodiments of the invention, the aqueous radioprotectant formulation comprises a sugar such as glucose, mannose, fructose, melizitose, N-acetyl-D-glucosamine, sucrose or trehalose. In some embodiments, the aqueous radioprotectant formulation comprises an antioxidant selected from the group consisting of ascorbate, urate, nitrite, vitamin E, α-tocopherol, and nicotinate methylester. In certain embodiments of the invention, the aqueous radioprotectant formulation comprises a buffering agent, for example, one selected from the group consisting of citrate, tris(hydroxymethyl)aminomethane (TRIS), and tartrate. In one embodiment, the radioprotectant formulation comprises sucrose and/or bicarbonate. The formulation may further comprise ascorbate (Asc) and paracetamol (PAM). An exemplary implementation is 25mM Asc, 10mM PAM, 50 mM sucrose, and 2M bicarbonate.

Various other protective excipients may be used. Reactive oxygen species (ROS) are antioxidant compounds that may also be used to provide protection. These include glutathione and superoxide dismutase. Hydroxyethyl acrylate may also be used as a protectant because it can scavenge free radical electrons. In certain embodiments, the aqueous radioprotectant formulation comprises hydroxyethyl acrylate in a concentration range from 1 mM to 50 mM. Other agents, such as polyethylene glycol (PEG) may also be included in these formulations.

In one or more embodiments, the aqueous radioprotectant formulation comprises a nitrate. In certain embodiments, the nitrate is sodium nitrate (NaNO₃). Sodium nitrate (NaNO3) is an oxidizer and scavenges free electrons caused by radiation processes, such as e-beam sterilization. It absorbs the electron and reduces into nitrate. In certain instances, the aqueous radioprotectant formulation comprises nitrate ion/sodium nitrate in a concentration range from 0.025 M to 1 M. Exemplary implementations include 100mM, 500mM, or 1M NaNO₃ with 50 mM sucrose and 2M bicarbonate. In one or more embodiments, other nitrates with a similar functionality as sodium nitrate may be used for e-beam protection and other radiation sterilization processes.

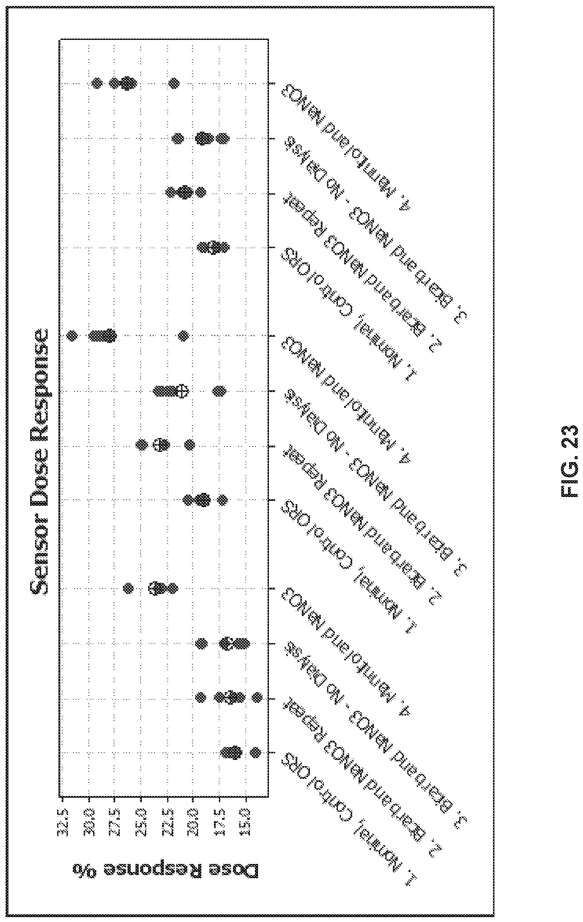

In another embodiment, the radioprotectant formulation further comprises mannitol. Mannitol is a sugar alcohol that is commonly used as a bulking agent, providing structure to freeze dried products. In certain instances, the aqueous radioprotectant formulation comprises mannitol in a concentration of at least 1 mM to 400 mM. An exemplary implementation is 100 mM NaNO₃, 50 mM sucrose, and 320 mM mannitol. Other bulking agents could also be used to add freeze dried structure to the sensor chemistry. Notably, illustrative experiments have found that replacing bicarbonate with mannitol in radioprotectant formulations increased sensor dose response by about 8% (see Figure 23).

Benzoyl peroxide is also an oxidizer and reactive oxygen species, and may be used as a protectant to e-beam radiation. In certain instances, the aqueous radioprotectant formulation comprises benzoyl peroxide in a concentration range from100 µM to 412 µM. Illustrative experiments have examined concentration of up to 412 µM for benzoyl peroxide as a protectant. Other moieties with radical electron forming capabilities are generally reactive oxygen species which will also act as e-beam protecting agents.

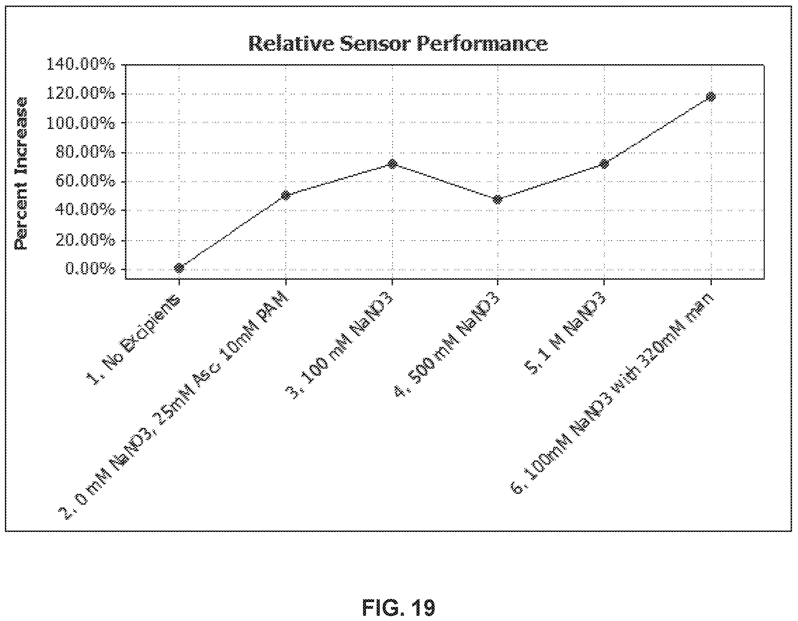

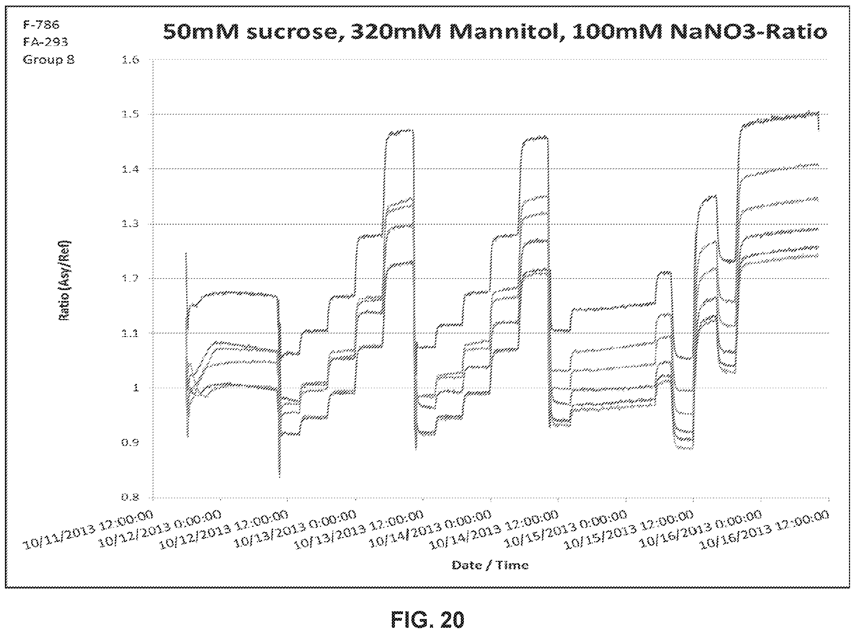

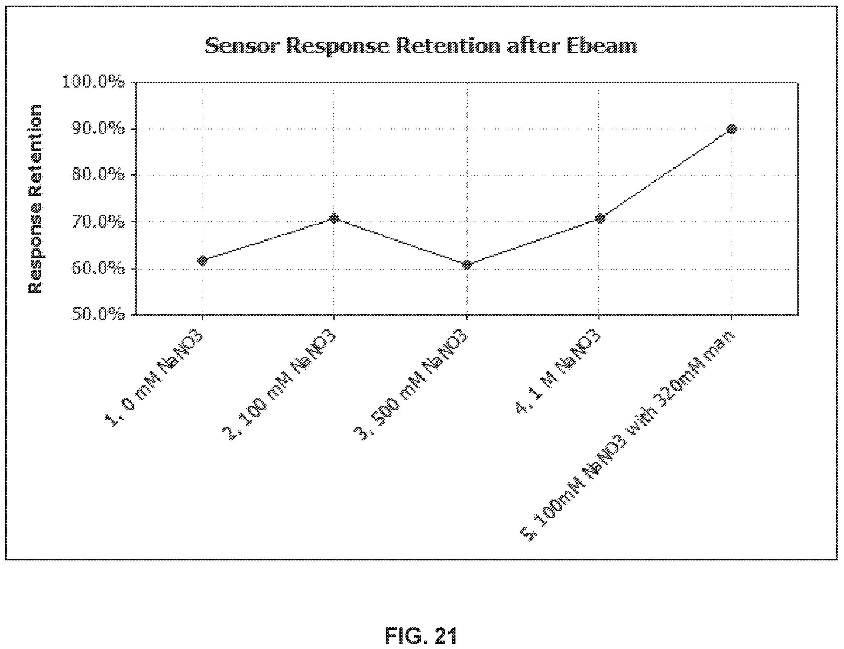

As shown for example in Figure 19, embodiments of radioprotectant formations as described herein are able to provide increased sensor performance when compared to sensors with no excipients. Illustrative experiments have demonstrated that the addition of sodium nitrate and/or other excipients improves the performance of sensor chemistry. Further, experiments have found that the sodium nitrate excipient is a more effective protectant than acetaminophen/paracetamol and ascorbate (see, e.g. Figure 19). Figure 20 is a graph illustrating the change of 50 mM sucrose, 320 mM mannitol, and 100 mM NaNO3 formation ratios over time. Further experiments have also demonstrated that the addition of sodium nitrate improves retention of dose response after e-beam, which is also an improvement over the protection offered by ascorbate and acetaminophen (see, e.g. Figure 21). The combination of sodium nitrate and mannitol was found to increase retention to 90%.

In typical methods of the invention, the sterilization process is performed under conditions selected to protect the functional integrity of the sterilized sensor. For example, in typical embodiments of the invention, the sterilization process is performed during or after cooling the device. In illustrative embodiments, the sterilization process is performed below a certain temperature or within a particular range of temperatures, for example below 10°C or below 5°C or at a temperature between 0 and 5°C, or between 0 and 10°C. In some embodiments of the invention, the sterilization process is performed under oxygen free conditions (e.g. when a formulation does not comprise an oxidizing compound). Optionally, the process is performed on a sensor within and aqueous formulation that has been de-aerated with argon gas, nitrogen gas, or the like. In some embodiments of the invention, the sterilization process is performed using a formulation having a pH below 7, below 6, or below 5 etc. In some embodiments of the invention, the sterilization process is performed under conditions selected so that the saccharide binds the saccharide binding polypeptide and/or the fluorophore quenching composition quenches the fluorophore so as to inhibit damage to the saccharide sensor that can result from the radiation sterilization process. Some methodological embodiments of the invention comprise further steps, for example those where an irradiated sensor composition comprising the aqueous radiation protecting formulation is dialyzed to alter the concentrations of one or more components in the formulation.

Another embodiment of the invention is a composition of matter comprising a saccharide sensor and a fluorophore. The saccharide sensing element of the saccharide sensor can comprise a boronic acid derivative, a molecular imprinted polymer or a polypeptide. In such compositions, the saccharide sensor is combined with a fluorophore quenching compound. One illustrative embodiment of the invention is a composition of matter comprising a saccharide sensor that includes a saccharide binding polypeptide having a carbohydrate recognition domain; and a fluorophore. In such compositions, the saccharide sensor is combined with an aqueous radioprotectant formulation comprising a saccharide, wherein the saccharide binds to the carbohydrate recognition domain. Optionally in such compositions, the saccharide sensor is also combined with a fluorophore quenching compound.

A number of compounds can be combined with the saccharide sensors disclosed herein to form the radioprotectant compositions of the invention. In typical embodiments of the invention, the composition comprises a saccharide selected from the group consisting of glucose, mannose, fructose, melizitose, N-acetyl-D-glucosamine, GluNac, sucrose or trehalose. In certain embodiment of the invention, the composition comprises a fluorophore quenching compound, for example, acetaminophen. In some embodiments of the invention, the composition comprises an antioxidant compound is selected from the group consisting of ascorbate, urate, nitrite, vitamin E, α-tocopherol or nicotinate methylester. In some embodiments of the invention, the composition comprises a surfactant, for example a polysorbate such as Tween 80. In certain embodiments of the invention, the composition comprises a buffering agent such as citrate, tris(hydroxymethyl)aminomethane (TRIS) or tartrate. Optionally the composition is formed to have a pH of 7 or below, 6 or below, or 5 or below.

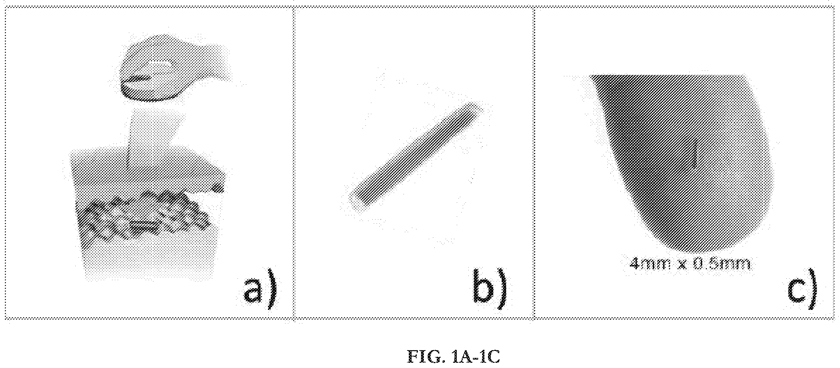

Specific compounds are observed to provide saccharide sensors (e.g. those shown in Figures 1A-1C) with high levels of protection against radiation damage when present in aqueous radioprotectant formulations in a particular concentration range. For example, in certain embodiments of the invention, the radiation protecting formulation comprises sodium nitrate in a concentration of at least 100 mM to 1 M (e.g. at least 100 mM, at least 200 mM, at least 300 mM, at least 400 mM, at least 500 mM, at least 600 mM, at least 700 mM, at least 800 mM, at least 900 mM etc.). Optionally, the radiation protecting formulation comprises mannitol in a concentration of at least 1 mM to 400 mM (e.g. at least 100 mM, at least 200 mM, at least 300 mM etc.). Optionally, the radiation protecting formulation comprises benzoyl peroxide in a concentration of at least 1 mM to 500 mM (e.g. at least 100 mM, at least 200 mM, at least 300 mM, at least 400 mM etc.). In certain embodiments of the invention, the radiation protecting formulation comprises acetaminophen in a concentration of at least 1 mM to 50 mM (e.g. at least 10 mM, at least 20 mM, at least 30 mM, at least 40 mM etc.). Optionally, the radiation protecting formulation comprises acetaminophen in a concentration of 20 mM ± 10 mM (and typically ± 5 mM). In certain embodiments of the invention, the radiation protecting formulation comprises sucrose in a concentration of at least 10 mM to 1000 mM (e.g. at least 100 mM, at least 200 mM, at least 300 mM, at least 400 mM etc.). Optionally, the radiation protecting formulation comprises sucrose in a concentration of 1 to 100 mM (e.g. at least 10 mM, at least 20 mM, at least 30 mM, at least 40 mM, at least 50 mM, at least 60 mM, at least 70 mM, at least 80 mM, at least 90 mM etc.). In specific embodiments, the radiation protecting formulation comprises sucrose in a concentration of 500 mM ± 200 mM (and typically ± 100 mM). In certain embodiments of the invention, the radiation protecting formulation comprises mannose in a concentration of at least 1 mM to 100 mM (e.g. at least 10 mM, at least 20 mM, at least 30 mM, at least 40 mM etc.). Optionally, the radiation protecting formulation comprises mannose in a concentration of 50 mM ± 20 mM (and typically ± 10 mM). In certain embodiments of the invention, the radiation protecting formulation comprises ascorbate in a concentration of at least 1 mM to 100 mM (e.g. at least 10 mM, at least 20 mM, at least 30 mM, at least 40 mM etc.). In certain embodiments of the invention, the radiation protecting formulation comprises ascorbate in a concentration of not more than 10 mM, 20 mM, 30 mM, 40 mM, 50 mM, 60 mM, 70mM, 80 mM, 90 mM or 100 mM. Optionally, the radiation protecting formulation comprises ascorbate in a concentration of 50 mM ± 20 mM (and typically ± 10 mM). In certain embodiments of the invention, the radiation protecting formulation comprises Tris in a concentration of at least 1 mM to 10 mM (e.g. at least 1 mM, at least 2 mM, at least 3 mM, at least 4 mM etc.). Optionally, the radiation protecting formulation comprises Tris in a concentration of 5 mM ± 2 mM (and typically ± 1 mM). In certain embodiments of the invention, the radiation protecting formulation comprises citrate in a concentration of at least 5 mM to 100 mM (e.g. at least 10 mM, at least 20 mM, at least 30 mM, at least 40 mM etc.). Optionally, the radiation protecting formulation comprises citrate in a concentration of 10 mM ± 2 mM (and typically ± 1 mM).

As shown by the working embodiments disclosed herein, one or more of these compounds is typically combined with another of these compounds in the radiation protecting formulations of the invention. For example, certain formulations of the invention will comprise sucrose combined with acetaminophen and/or ascorbate and/or Tris and/or citrate. Similarly, certain formulations of the invention will comprise acetaminophen combined with sucrose and/or ascorbate and/or Tris and/or citrate. Similarly, certain formulations of the invention will comprise ascorbate combined with sucrose and/or acetaminophen and/or Tris and/or citrate. Similarly, certain formulations of the invention will comprise citrate combined with sucrose and/or acetaminophen and/or Tris and/or ascorbate. The formulations can comprise additional compositions such as one or more amino acids or pharmaceutically acceptable salts, for example those disclosed in Remington: The Science and Practice of Pharmacy, University of the Sciences in Philadelphia (Ed), 21st Edition (2005). As the sensor is to be used in the body, in typical embodiments, the excipients are commonly acceptable for use in the body.

As noted above, embodiments of the invention disclosed herein provide methods and materials useful in sterilization procedures for medical devices such as glucose sensors. While glucose sensors are the common embodiment discussed herein, embodiments of the invention described herein can be adapted and implemented with a wide variety of medical devices. As discussed in detail below, typical sensors that benefit from the methods and materials of the invention include, for example, those having sensing complexes that generate an optical signal that can be correlated with the concentration of an analyte such as glucose. A number of these sensors are disclosed, for example in U.S. Patent Application Publication Nos. 20080188723, 20090221891, 20090187084 and 20090131773. Embodiments of the invention described herein can also be adapted and implemented with amperometric sensor structures, for example those disclosed in U.S. Patent Application Publication Nos. 20070227907, 20100025238, 20110319734 and 20110152654,.

The compositions used in embodiments of the invention exhibit a surprising degree of flexibility and versatility, characteristics which allow them to be adapted for use in a wide variety of sensor structures. In some embodiments of the invention, one or more sensor elements can comprise a structure formed from a polymeric composition through which water and other compounds such as analytes (e.g. glucose) can diffuse. Illustrative polymeric compositions are disclosed in U.S. Patent Publication No. 20090221891 and include, for example, material (e.g. one that is biodegradable) comprising a polymer having hydrophobic and hydrophilic units. Specific polymers can be selected depending upon a desired application. For example, for mobility of glucose, a material can be selected to have a molecular weight cut-off limit of no more than 25000 Da or no more than 10000 Da. Components disposed within such polymeric materials (e.g. sensing complexes) can be of high molecular weight, for example proteins or polymers, in order to prevent their loss from the sensor by diffusion through the polymeric materials. In an illustrative embodiment, hydrophilic units of a polymeric material comprise an ester of polyethylene glycol (PEG) and a diacid, and the molecular weight cut-off limit is affected by the PEG chain length, the molecular weight of the polymer and the weight fraction of the hydrophilic units. The longer the PEG chains, the higher the molecular weight cut-off limit, the higher the molecular weight of the polymer, the lower the molecular weight cut-off limit, and the lower the weight fraction of the hydrophilic units, the lower the molecular weight cut-off limit.

Sensor components can be selected to have properties that facilitate their storage and or sterilization. In some embodiments of the invention, all components of the sensor are selected for an ability to retain sensor function following a sterilization procedure (e.g. e-beam sterilization). In some embodiments of the invention, all components of the sensor are selected for an ability to retain sensor function following a drying procedure (e.g. lyophilization).

Optionally the sensing complex produces an optical signal that can be correlated with an analyte of interest, for example, glucose. A sensing complex (e.g. one comprising a binding assay) generating the optical signal should typically be reversible such that a continuous monitoring of fluctuating levels of analyte can be achieved. Optionally, the detectable or measurable optical signal is generated using a proximity based signal generating/modulating moiety pair so that a signal is generated or modulated when a first member of the pair is brought into close proximity with a second member of the pair. In one illustrative embodiment, the analyte binding agent (e.g. a lectin such as mannose binding lectin as disclosed in WO 2006/061207) is labelled with one of a proximity based signal generating/modulating moiety pair and the analyte analogue is labelled with the other of the proximity based signal generating/modulating moiety pair, and there is a detectable difference in signal when the analyte analogue and analyte binding agent form the complex and when the analyte analogue is displaced by the analyte from the complex. Typically, the proximity based signal generating/modulating moiety pair is an energy donor moiety and energy acceptor moiety pair. Energy donor moieties and energy acceptor moieties are also referred to as donor and acceptor chromophores (or light absorbing materials) respectively. An energy acceptor which does not emit fluorescence is referred to as a quenching moiety. In such embodiments, a lectin can be labelled with one of an energy donor and energy acceptor moiety pair and the analyte analogue is labelled with the other of the energy donor and energy acceptor moiety pair. The detectable difference in signal corresponds to a detectable difference in energy transfer from the energy donor moiety to the energy acceptor moiety. Optionally, the analyte analogue bears the energy acceptor moiety and the analyte binding agent bears the energy donor moiety. In certain embodiments of the invention, the sensor of the invention incorporates an assay which generates an optical readout using the technique of fluorescence resonance energy transfer (FRET).

In one illustrative embodiment of the sensors discussed in the paragraph above, the variants of the competitive binding assay each comprise: an analyte binding agent labelled with a first light-absorbing material; a macromolecule labelled with a second light-absorbing material and comprising at least one analyte analogue moiety; wherein the analyte binding agent binds at least one analyte analogue moiety of the macromolecule to form a complex from which said macromolecule is displaceable by said analyte, and wherein said complex is able to absorb light energy and said absorbed light energy is able to be non-radiatively transferred between one of the light-absorbing materials and the other of the light-absorbing materials with a consequent measurable change in a fluorescence property of said light absorbing materials when present in said complex as compared to their said fluorescence property when said macromolecule is displaced by said analyte from said complex, and wherein the different variants of the assay are distinguished by the number of analyte analogue moieties present in the macromolecule. Such sensors are disclosed, for example in U.S. Patent Application Publication Nos. 20080188723, 20090221891, 20090187084 and 20090131773.

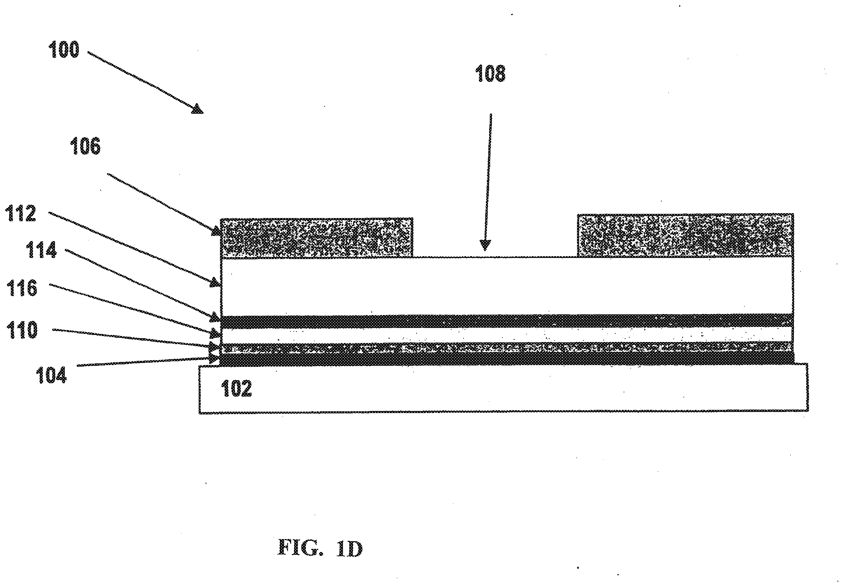

In other embodiments of the invention, the sensor comprises planar layered elements and, for example comprises a conductive layer including an electrode, an analyte sensing layer disposed over the conductive layer (e.g. one comprising glucose oxidase); and an analyte modulating layer disposed over the analyte sensing layer. In certain embodiments of the invention, the sensor electrode is disposed within a housing (e.g. a lumen of a catheter). The sensor embodiment shown in Figure 1D is a amperometric sensor 100 having a plurality of layered elements including a base layer 102, a conductive layer 104 which is disposed on and/or combined with the base layer 102. Typically the conductive layer 104 comprises one or more electrodes. An analyte sensing layer 110 (typically comprising an enzyme such as glucose oxidase) is disposed on one or more of the exposed electrodes of the conductive layer 104. A protein layer 116 disposed upon the analyte sensing layer 110. An analyte modulating layer 112 is disposed above the analyte sensing layer 110 to regulate analyte (e.g. glucose) access with the analyte sensing layer 110. An adhesion promoter layer 114 is disposed between layers such as the analyte modulating layer 112 and the analyte sensing layer 110 as shown in Figure 1D in order to facilitate their contact and/or adhesion. This embodiment also comprises a cover layer 106 such as a polymer coating can be disposed on portions of the sensor 100. Apertures 108 can be formed in one or more layers of such sensors. Amperometric glucose sensors having this type of design are disclosed, for example are disclosed, for example, in U.S. Patent Application Publication Nos. 20070227907, 20100025238, 20110319734 and 20110152654.

Embodiments of the invention can be used with sensors having a variety of configurations and/or sensing complexes. In certain methodological embodiments of the invention, the sensor comprises a cylindrical polymeric material, the internal matrix comprises an encapsulated longitudinal cavity, and the sensing complex comprises a carbohydrate binding lectin (e.g. mannose binding lectin which binds glucose) coupled to a fluorophore pair. In other methodological embodiments of the invention, the sensor comprises an electrode coated with glucose oxidase and a glucose limiting membrane disposed over the glucose oxidase, wherein the glucose limiting membrane modulates the diffusion of glucose therethrough.

### EXAMPLES

The Examples below provide illustrative methods and materials that can be used in the practice of the invention.

### Example 1: Illustrative Methods and Materials for Use with Embodiments of the Invention

Sterilization of medical devices is important and the choice of sterilization method is based on which methods would be both safe and least destructive to the medical device. Three methods of sterilization are commonly used with medical devices. These are heat sterilization, gas sterilization and radiation sterilization. Heat sterilization can be problematical for devices that include proteins because the heat can denature the proteins (protein unfolding happens at approx. 60 °C). Gas sterilization process can be difficult to use in medical devices that end up as a wet device because getting a gas into even small amounts of liquid (and out again) can be difficult. For these reasons radiation sterilization is a method of choice for use with many devices such as the glucose sensors discussed herein. Moreover, as e-beam is typically easier to control than gamma radiation, e-beam radiation is used in the illustrative examples disclosed herein. As noted below, e-beam radiation of protein containing solutions can lead to a loss of protein activity in these sensors. In addition, e-beam radiation of dyes can lead to bleaching of the dyes. Both these effects can contribute to losses in sensor activity.

In aqueous solutions, the radiolysis of water can initiate oxidation reactions of compounds dissolved in water. The treatment of aqueous solutions by electron beam irradiation can decrease the concentration of certain compounds, provided that the energy absorbed (dose) is sufficient.

During radiolysis (e.g. electron beam; eb) H₂O turns into the following species (brackets show the formation of species in µmoles/J):

OH•, eaq, H•, H₃O⁺, H₂, H₂O₂

H₂O + eb → [0.28]OH• +[0.27]e-(aq) + [0.6]H• + [0.07]H₂O₂ + [0.27]H₃O⁺ + [0.05]H2

These entities formed by the radiolysis of water initiate many reactions with compounds present and in literature phenol degradation is often used as model compound to study the effect of the radiolysis.

The ionization of the assay components themselves in the solution is minimal compared to the radiolysis of the aqueous solvent since the concentration of assay is in the range of µM and the concentration of water will be approx. 55 M, i.e. the damaging effects of electron beam radiation to the assay origins from attack from water radiolysis products.

In the optical sensor assay the protein appears in the concentration of µM i.e. water is present is 10⁷ times the concentration of protein.

As discussed in detail below, a number of compounds were identified and tested to assess their ability to protect sensors against radiation damage.

### Protection of Polymers

Embodiments of the invention are designed to protect sensors that comprise polymers such as Poly Active™. Poly Active™ is a biodegradable polymeric drug delivery system. Poly Active represents a series of poly(ether ester) multiblock copolymers, based on poly(ethylene glycol), PEG, and poly(butylene terephthalate), PBT.

Polymers such as Poly Active™ can be protected against radiation damages by the presence of α-tocopherol. The α -tocopherol is added to the polymer by the manufacturer and is an antioxidant (Vitamin E) often used to protect products against radiation damage. In the Poly Active polymer used in the optical sensor it is expected that the α -tocopherol predominantly will be in the lipophilic domains of the polymer.

### Decoloration of dyes

Embodiments of the invention are designed to protect sensors that comprise dyes such as Alexa Fluor® fluorescent dyes. Decoloration of dye containing water, happens when the extensive electron conjugated system of the dye molecules is destroyed. The presence of radicals in the solution can initiate this process.

### Protein degradation

Embodiments of the invention are designed to protect sensors that comprise proteins such as MBL. Radiation damages to proteins are most often initiated by the damage of the disulphide bond RSSR formed by the cysteine residues. Cysteine amino acids are the most affected amino acid by radiation. Radiation damages occur when disulfide bridges break and carbonyl groups of acidic residues lose their definition thus causing the proteins to lose their activity.

The MBL protein has cysteine rich N-terminal domains (see, e.g. NCBI Reference Sequence: NP_000233.1). The tertiary structure of MBL is maintained by the RSSR bridges in the N-Terminal and if these are broken the structure of the protein and hence the function of the protein is lost. Wallis et al., J Biol Chem 274: 3580 (1999) shows a schematic of a polypeptide unit of MBL. In order to protect the protein from radiation damages one can endeavor to protect the cysteine residues of the N-Terminal and the CRD's.

### Protection against radiation damages

Art teaches that the prime species that damages proteins and other molecules in solution is the OH• (hydroxyl radical) hence this is the species to look for during protection. Antioxidants such as ascorbate can be used to protect proteins from damages by ionizing radiation. Prior art shows that the concentration of ascorbate used to protect the proteins is 0.2 M or higher, most likely due to the need for continuous antioxidant protection.

Antioxidants (e.g. ascorbate) have been described in literature for use in radiation protection of dyes. Vahdat et al., Radiation Physics and Chemistry 79 (2010) 33-35 reports that electron beam irradiation induced oxidation leading to decoloration and decomposition of the dye C.I. Direct Black 22. Holton, J. Synchotron Rad. (2009), 16, 133-142 reports that ascorbate, nicotinic acid, DNTB, nitrate ion, 1,4-benzoquinone, TEMP and DTT have a protective effect against radiation damage to protein crystals. Wong et al., Food Chemistry 74 (2001) 75-84 reports the effect of L-ascorbic acid (LAA) on oxidative damage to lipid (linoleic acid emulsion) caused by electron beam radiation.

### Ascorbate action

The mechanism of action of protectants is to, for example, scavenge the radicals formed by radiolysis. The ascorbate is capable of reducing the hydroxyl radical. The ascorbate radical will undergo several processes e.g. disproportionately to ascorbate and dehydro-ascorbate (DHA). Due to this possible mode of action (ascorbate radical acting both as oxidizer and reducer) too high a concentration of ascorbate could be damaging to the chemistry of certain sensor embodiments.

### Acetaminophen action

Acetaminophen is easily oxidized in aqueous solution and hence is able to reduce radicals in solution. Since this compound also works as a fluorescence quencher for the AF594 donor fluorophore and AF700 reference fluorophore in a glucose assay system with these components, it appears that acetaminophen protects the dyes from bleaching due to its presence near the lipophilic areas of both the protein and the dyes.

Acetaminophen is more lipophilic than ascorbate and could hence act as a lipophilic radical scavenger primarily protecting the vulnerable domains (RSSR bridges and aromatic systems of the dyes) close to lipophilic domains in the compounds needing protection. This predominant lipophilic protection from acetaminophen combined with ascorbate's high solubility in aqueous solution protecting the more hydrophilic domains can be a powerful combination when looking for protection.

### Sucrose and Mannose

Polyols like mannitol may be good radical scavenges and hence such carbohydrates also could yield some protection against radiation damages (hydrophilic domains). Further sucrose is known to have a stabilizing effect on the MBL hence this could help to improve the storage stability of the assay and mannose would bind to the CRD and create some stabilization effect here. Indeed carbohydrates add protective effects to the assay.

### Buffer System

Amine containing buffer systems like Tris and HEPES are known to provide some protection to the proteins. Especially they provide protection against tryptophan loss from proteins. Protective effects from Tris buffer were also observed. Using Citrate as part of the buffer system keeps pH around 6 during storage. Citrate is a tertiary alcohol and alcohols like t-butanol (a tertiary alcohol) and isopropyl alcohol (a secondary alcohol) is known scavengers for radiolysis radicals.

### Example 2: Radiation Dose

In initial e-beam experiments, sterilization at a 15 kGy dose was used for the optical glucose sensor, one that comprises both MBL and fluorophore compositions. The conclusion was to further identify and test excipients first for their individual protection capability and later take the best from each class and use them in combination.

The following experiments were all conducted with radiation dose of 15 kGy while the sensors were cooled and oxygen free (except when the excipient was an oxidizing compound). After radiation the performance of the sensors was evaluated. The primary parameters evaluated as being retained was the Dose Response (DR relative to 0 kGy DR) as well as the absolute DR (measured in degrees phase shift from 40 mg/dL glucose to 400 mg/dL glucose) after the 15 kGy radiation dose. Also, sensor signal drift after radiation was observed but not quantified. The first initial experiments with sterilizing unformulated sensors (control sensors not combined with any radioprotectant compositions) yielded the results shown in Figure 2. Figure 2 shows a graph of data from experiments observing a retained dose response for unformulated sensors as a function of e-beam doses. The triple dose is 3×5 kGy. The sensors tested were radiated wet in a solution comprising 50 mM Tris-buffer saline.

A dose of 15 kGy as target for the radiation dose is a reasonable choice as there is still 50% retention of DR after irradiation of the unformulated fluorescent sensors. In addition the electrochemical sensors discussed herein are irradiated with 16 kGy if they have a low bioburden after production (<1.5 cfu). Due to the simplicity of the production of the optical sensor, it is expected for this low bioburden to be the rule (and not the exception). Hence, a 15 kGy dose of e-beam is expected to provide sterility.

### Example 3: Tests of excipients useful to protect fluorescent sensors from

### radiation damage

Experiments were conducted on the fluorescent glucose sensor shown in Figures 1A-1C, one comprising MBL and fluorophore compounds (see, e.g. U.S. patent application publication 2008/0188723). Excipients used for protecting the sensor during e-beam sterilization processes were consequently chosen to protect MBL and these fluorophores. Dextran was considered to benefit from the protection applied to MBL. The protective excipients were chosen from the following categories:
*Known MBL Binding Sugars:* Binding sugars can protect the carbohydrate recognizing domain (CRD) of the protein, by keeping the peptide structure in the right conformation. However this is not thermodynamically favored compared to non-binding sugars. ΔG = ΔH - TΔS. For binding sugars the TΔS contribution is large due to the binding sugar in the CRD being in an ordered conformation instead of the random (non-ordered) water structure in the CRD. The binding sugar will then lower the loss in ΔG less than a low binding sugar due to entropy effects.
*Low-Binding Sugars:* Low-binding sugars can function to provide a more rigid hydrogen-bonding scaffold (compared to water) to support the structure of the protein during radiation.
*Antioxidants:* Antioxidants are generally used as protective agents against free radical associated radiation damage. Antioxidants quench radicals by reducing them.
*Oxidants:* Oxidants were tested as protective agent for the reduction of the fluorescent dyes. Radicals generated during irradiation could reduce the dyes resulting in bleaching them. Oxidants could oxidize the dye-radicals formed thus protecting the dyes. Further in this context these compounds were trialed also to show the benefit of using antioxidants.
*Amino Acids:* Amino acids are often used to stabilize pharmaceutical formulations. Both hydrophilic and hydrophobic amino acids were tested.
*Surfactants:* Surfactants are often used to stabilize pharmaceutical formulations since denaturing often happens at phase transitions or boundaries.
*Phenyl Compounds:* Phenyl containing compounds may stabilize the fluorescent dyes via a π-π stacking mechanism (and hence the assay).
*Bacteriostats:* Bacteriostat compounds tested were phenyl containing compounds.

In the following experiments, two or more excipients were chosen from each category and tested individually and in combination with ascorbate. For the best excipients in four categories a larger matrix of experiments was trialed.

### Results from screening round

The list of excipients tested and the concentration of each is shown in Table 1 below.

**Table 1. A list of tested excipients to protect our sensors during radiation. All excipients were dialyzed into the sensor prior to irradiation. The sensors formulated with oxidative excipients were not de-aerated prior to radiation all other were de-aerated with Ar.**

| **Excipient Type** | **Excipients Used** | **Concentration range tested** | **Used in combinations¹⁾** |
|---|---|---|---|
| Binding Sugars | Mannose | 1, 2, 5, 10, 20 and 50 mM | Yes |
| | Fructose | 50 mM | No |
| | Melizitose | 20 mM | No |
| Low-Binding Sugars | Sucrose | 100, 500 and 1000 mM | Yes |
| | Trehalose | 500 and 1000 mM | Yes |
| Antioxidants | Ascorbate | 5, 50, 100 and 250 mM | Yes |
| | Nitrite | 5, 10 and 20 mM | Yes |
| | Ureate | 1 and 5 mM | Yes |
| | α -Tocopherol | 1 mg/mL (4.6 mM) | Yes |
| | Nicotinate methylester | 20 and 50 mM | No |
| Oxidants | H₂O₂ | 50 mM | Yes |
| | N₂O | Sat'd (gas bubbled through) | No |
| Amino Acids | Lysine | 2 mg/mL | No |
| | Tryptophan | 2 mg/mL | No |
| | Phenylalanine | 2 mg/mL | No |
| Surfactants | Synperonic | 1 mg/mL | No |
| | Tween 20 | 1 mg/mL | No |
| | Tween 80 | 1 mg/mL | No |
| "Drugs" | Acetaminophen | 1, 2, 5, 10 and 20 mM | Yes |
| | Acetylsalicylic acid | 10 mM | Yes |
| | α -Tocopherol | 1 mg/mL (4.6 mM) | Yes |
| Phenyl Containing Compounds | Acetaminophen Acetylsalicylic acid | 1, 2, 5, 10 and 20 mM | Yes |
| | | 10 mM | Yes |
| | α -Tocopherol | 1 mg/mL (4.6 mM) | Yes |
| | Phenol | 1 mg/mL (106 mM) | Yes |
| | m-Cresol | 1 mg/mL (92 mM) | Yes |
| | Tryptophan | 2 mg/mL (98mM) | Yes |
| | Phenylalanine | 2 mg/mL | No |
| | Nicotinate methylester | 20 and 50 mM | No |
| Bacteriostats | Phenol | 1 mg/mL (106 mM) | Yes |
| | m-Cresol | 1 mg/mL (92 mM) | Yes |
| Combinations | +80 | Max molarity 1 M | |

| | | | |
|---|---|---|---|
| ¹⁾ The combination most often used was together with ascorbate. | | | |

The excipients listed in Table 1 were evaluated in order to choose which compounds should be used for the test of different combination of excipient. Test endeavored to identify compounds that individually had an expected protective property towards a preferred target (*e*.*g*. CRD, Dye, General peptide bond or protein and storage stabilizing effects).

Table 2 provides a brief summary of the results of the screening round. In Table 2, an overview of the excipients tested as protective agents against radiation damages during e-beam (15 kGy dose) is provided. The excipients are listed according to class of compound. Some of the excipients are listed in more than one category.

**Table 2**

| **Excipient Type** | **Excipients** | **Retention Range (with acc. DR)** | **Best In Class** |
|---|---|---|---|
| Binding Sugars | Mannose | 47% - 55% | Mannose |
| | Fructose | | |
| | Melizitose | | |
| Non-Binding Sugars | Sucrose | 47% - 90% | Sucrose |
| | Trehalose | | |
| Antioxidants | Ascorbate Nitrite Ureate α- Tocopherol Nicotinate methylester | 28% - 80% | Ascorbate |
| Oxidants | H₂O₂ | 38% - 58% | N₂O |
| | N₂O | | |
| Amino Acids | Lysine Tryptophan Phenylalanine | 44% - 95%¹⁾ | Tryptophan |
| Surfactants | Synperonic Tween 20 Tween 80 | 26% - 33% | Synperonic |
| "Drugs" | Acetaminophen Acetylsalicylic acid α -Tocopherol | 10% - 80% | Acetaminophen |
| Phenyl Containing Compounds | Acetaminophen Acetylsalicylic acid α -Tocopherol Phenol m-Cresol Tryptophan Phenylalanine Nicotinate methylester | 10% - 80% | Acetaminophen |
| Bacteriostats | Phenol m-Cresol | 0% | N/A |
| Combinations | +80 | 50 - +80% | |

From Table 2 the following four excipients (all best in their excipient class) were chosen to be used in combination as follows:
*Ascorbate*: Used for general protection of the peptide bonds in proteins. In literature mentioned as the best antioxidant and yielding best protection of proteins against free radical attack. However in literature the best protection is obtained with very high concentrations of ascorbate, most often > 200 mM which is at least four times the best concentration identified herein. Surprisingly, in tests of the sensor embodiments disclosed herein, it was found that using high concentrations of ascorbate (e.g. 250 mM) yields poor protection while low concentrations of ascorbate (e.g. not more than 100 mM, not more than 50 mM etc.) yields good protection.
*Acetaminophen*: This compound is not known to interfere with the protein in the assay. However it works as a dynamic and reversible quencher of the fluorescence from AF594. This means that acetaminophen has an effect on the AF594 and could help to protect the dye from radiation damages, e.g. prevent bleaching.
*Mannose*: Mannose could protect the carbohydrate recognizing domain (CRD) of the protein, by keeping the peptide structure in the right conformation.
*Sucrose*: Sucrose is often used for building a more rigid hydrogen-bonding scaffold (compared to water) to support the structure of the protein during radiation. Also sucrose could bring some improved storage stability to the assay.

The list of combinations with the concentration of each excipient and the results are shown in Table 3: Table 3 shows 48 variations over the four chosen excipients that have been tested. The order of the variations is stochastic.

**Table 3**

| **Excipient concentration (mM)** | | | | **Dose response** | | |
|---|---|---|---|---|---|---|
| Ascorbate | Acetaminophen | Mannose | Sucrose | 0 kGy | 15 kGy | Retained¹⁾ |
| 50 | 20 | 5 | | 1.6 | 1.8 | 112.5% |
| 50 | 20 | 5 | 500 | 1.1 | 1.6 | 145.5% |
| *50 | 20 | 1 | | 1.8 | 2.1 | 116.7% |
| 50 | 20 | 1 | 100 | 2.2 | 1.9 | 86.4% |
| *50 | 20 | 1 | 500 | 1.5 | 2.1 | 140.0% |
| 50 | 10 | 5 | | 1.8 | 1.7 | 94.4% |
| 50 | 10 | 5 | 100 | 1.5 | 1.8 | 120.0% |
| 50 | 10 | 5 | 500 | 2.0 | 1.6 | 80.0% |
| 50 | 10 | 1 | | 1.5 | 1.0 | 66.7% |
| 50 | 10 | 1 | 100 | 1.8 | 1.4 | 77.8% |
| 50 | 10 | 1 | 500 | 1.7 | 1.0 | 58.8% |
| 50 | 10 | | | 1.9 | 1.7 | 89.5% |
| 50 | 5 | 5 | | 0.8 | 1.7 | 212.5% |
| 50 | 2 | | | 2.0 | 1.1 | 55.0% |
| 50 | | 5 | | 2.1 | 1.7 | 81.0% |
| 50 | | 5 | 100 | 1.9 | 1.4 | 73.7% |
| 50 | | 5 | 500 | 1.7 | 1.8 | 105.9% |
| 50 | | 1 | | 1.8 | 1.7 | 94.4% |
| 50 | | 1 | 100 | 1.7 | 1.5 | 88.2% |
| 50 | | 1 | 500 | 1.8 | 1.8 | 100.0% |
| 50 | | | 1000 | 2.3 | 1.8 | 78.3% |
| 20 | 10 | | | 2.0 | 1.7 | 85.0% |
| 10 | 20 | 5 | | 1.6 | 1.0 | 62.5% |
| 10 | 20 | 5 | 100 | 0.8 | 0.8 | 100.0% |
| 10 | 20 | 5 | 500 | 2.2 | 1.2 | 54.5% |
| 10 | 20 | 1 | | 1.9 | 1.9 | 100.0% |
| 10 | 20 | 1 | 100 | 0.9 | 0.8 | 88.9% |
| 10 | 20 | 1 | 500 | 2.1 | 1.7 | 81.0% |
| 10 | 10 | 5 | | 1.3 | 1.5 | 115.4% |
| 10 | 10 | 5 | 100 | 1.6 | 1.7 | 106.3% |
| 10 | 10 | 5 | 500 | 1.7 | 1.6 | 94.1% |
| 10 | 10 | 1 | | 1.7 | 1.5 | 88.2% |
| 10 | 10 | 1 | 100 | 1.7 | 1.0 | 58.8% |
| 10 | 10 | 1 | 500 | 2.0 | 1.9 | 95.0% |
| 10 | 5 | 5 | | 1.9 | 1.3 | 68.4% |
| 10 | 2 | | | 1.8 | 1.0 | 55.6% |
| 10 | | 5 | 100 | 1.8 | 1.6 | 88.9% |
| 10 | | 1 | | 1.0 | 0.0 | 0.0% |
| 10 | | 1 | 100 | 1.8 | 1.5 | 83.3% |
| 5 | 2 | | | 1.8 | 1.2 | 66.7% |
| 5 | | | 1000 | 2.3 | 1.8 | 78.3% |
| | 20 | | | 2.2 | 1.7 | 77.3% |
| | 10 | | | 2.0 | 1.6 | 80.0% |
| | 5 | | | 2.2 | 1.6 | 72.7% |
| | 2 | | | 2.3 | 1.5 | 63.0% |
| | 1 | | | 2.4 | 1.3 | 54.2% |
| | | | 100 | 2.8 | 1.7 | 60.7% |
| | | | 500 | 1.8 | 1.9 | 105.6% |

| | | | | | | |
|---|---|---|---|---|---|---|
| ¹⁾ Retained DR >100% should not be possible but if the 0 kGy DR is unexpected low retained DR can become >100% * High absolute DR after radiation | | | | | | |

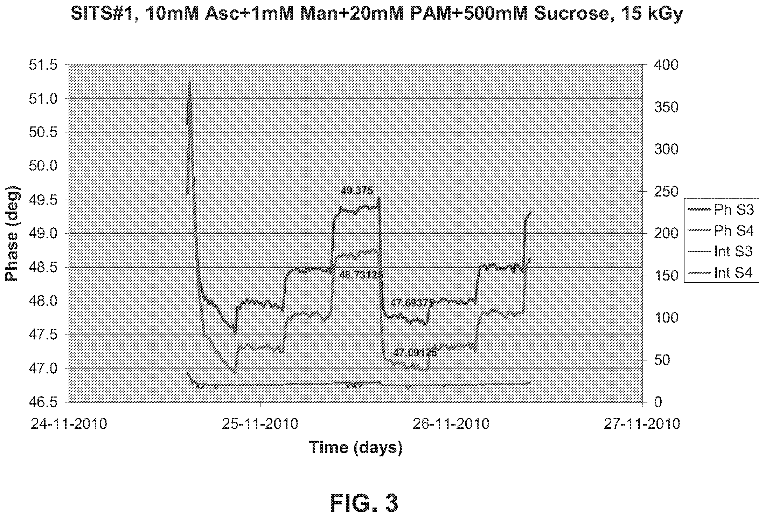

The plot of data shown in Figure 3 from the SITS system shows a test run of a set of sensors that has had good protection during radiation. Figure 3 shows a plot of phase and intensity data obtained from sensors after exposure to 15 kGy of radiation. The dose response is 1.7 after radiation compared to 2.1 before *i.e*. a retention of 81%. Note the long equilibration time of the sensor after startup. This most likely origins from the large concentration of sucrose used in the formulation. As is known in the art, concentrations of agents in aqueous solutions can be easily changed via processes such as dialysis.

### Excipients individual effects

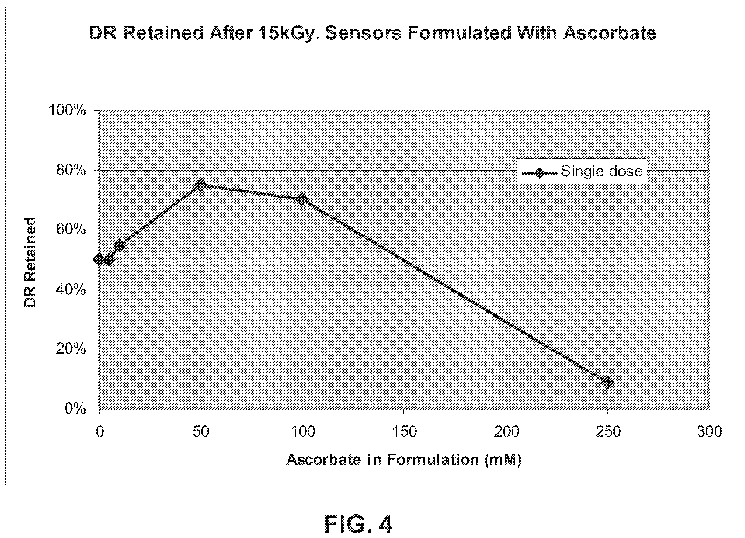

In order to get an overview of the effect of the individual excipients the results were visualized as seen Figure 4. Figure 4 shows a graph of data on DR retained for irradiated sensors as a function of ascorbate concentration used for formulation. Too low or too high concentrations of ascorbate used both yield low retained DR whereas the 20 mM to 100 mM concentration range yields good protection.

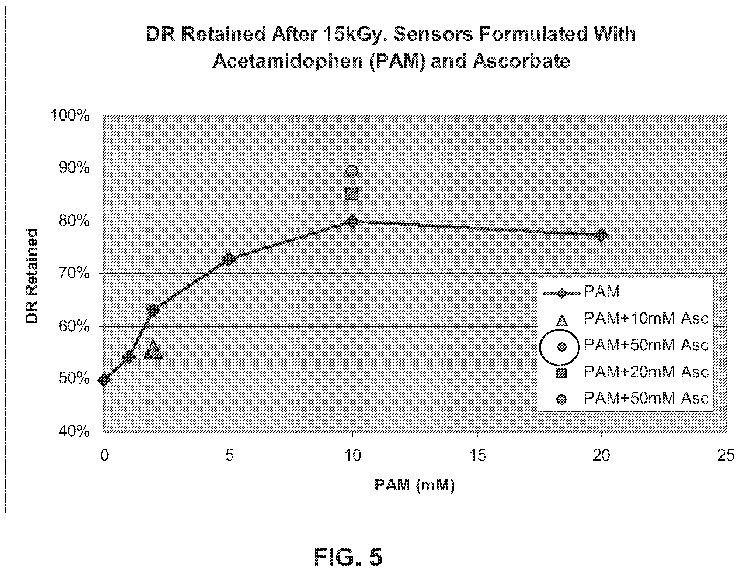

Figure 5 shows a graph of data on DR retained for irradiated sensors as a function of acetaminophen (=paracetamol, hence abbreviated PAM) concentration used for formulation. It is seen that using low concentrations of acetaminophen yields low retained DR whereas the use of concentrations above 10 mM yields good protection. Further it is shown that adding ascorbate to the excipients in most cases gives better protection.

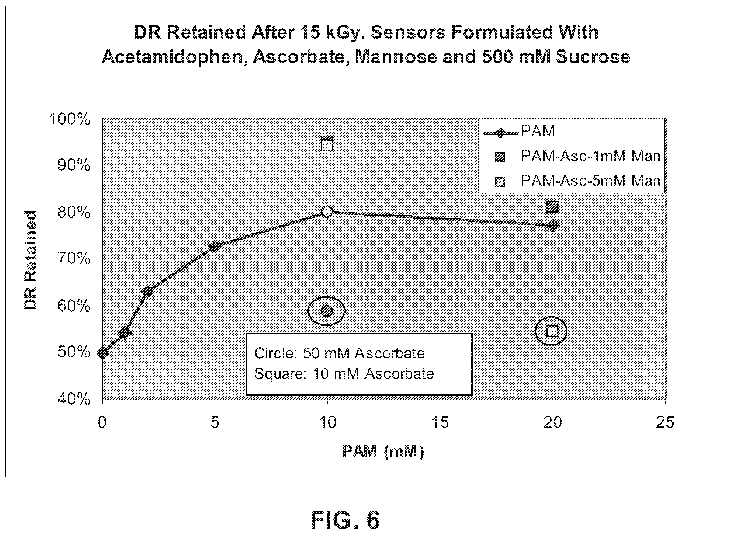

Figure 6 shows data of DR retained for irradiated sensors as a function of acetaminophen concentration used for formulation.

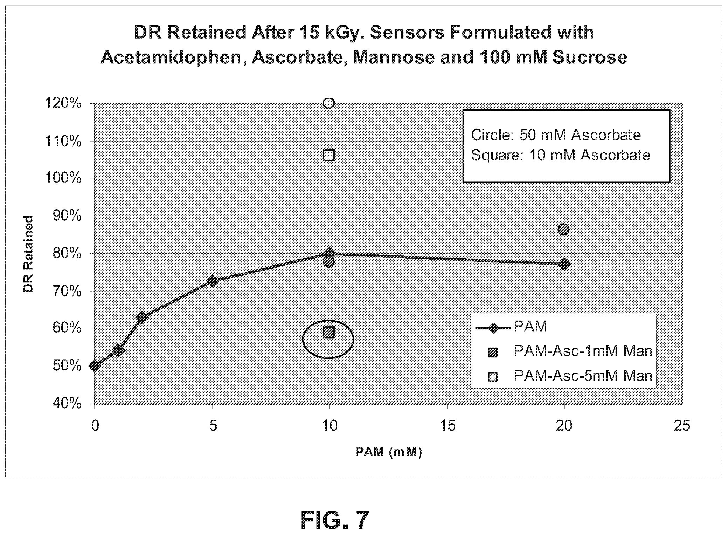

Figure 7 shows data of DR retained for irradiated sensors as a function of acetaminophen concentration used for formulation. All sensors have contained 100 mM sucrose and variation of additions of ascorbate and mannose are also shown.

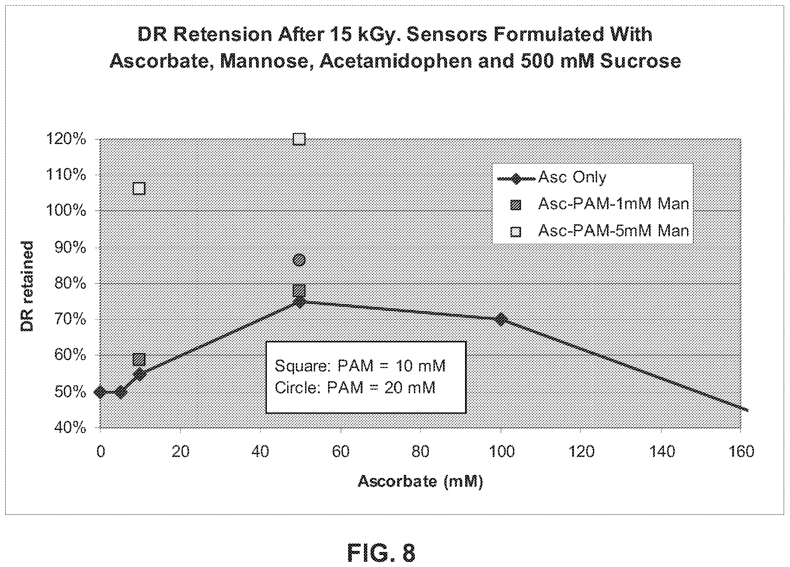

Figure 8 shows data of DR retained for irradiated sensors as a function of ascorbate concentration used for formulation. All sensors have contained 500 mM sucrose and variation of additions of acetaminophen (PAM) and mannose are also shown.

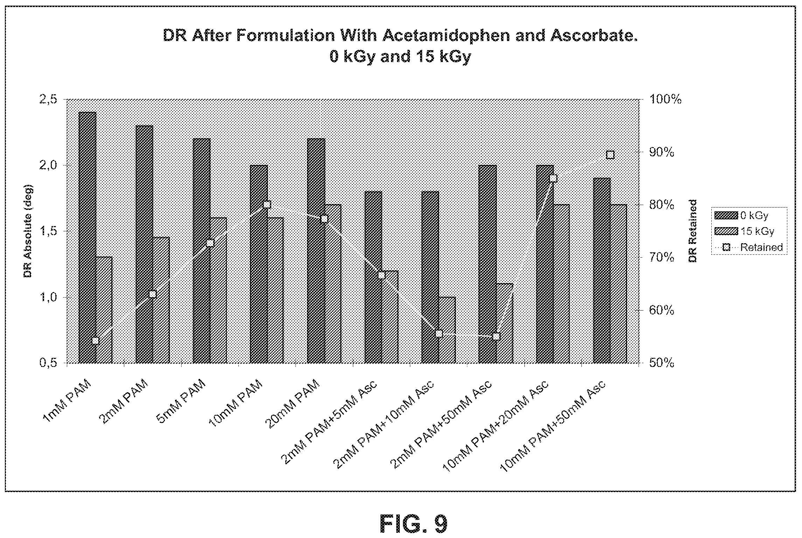

Figure 9 shows a bar graph of data presenting the absolute DR for both radiated and non-radiated sensor as a function of formulating the sensors with acetaminophen and ascorbic acid.

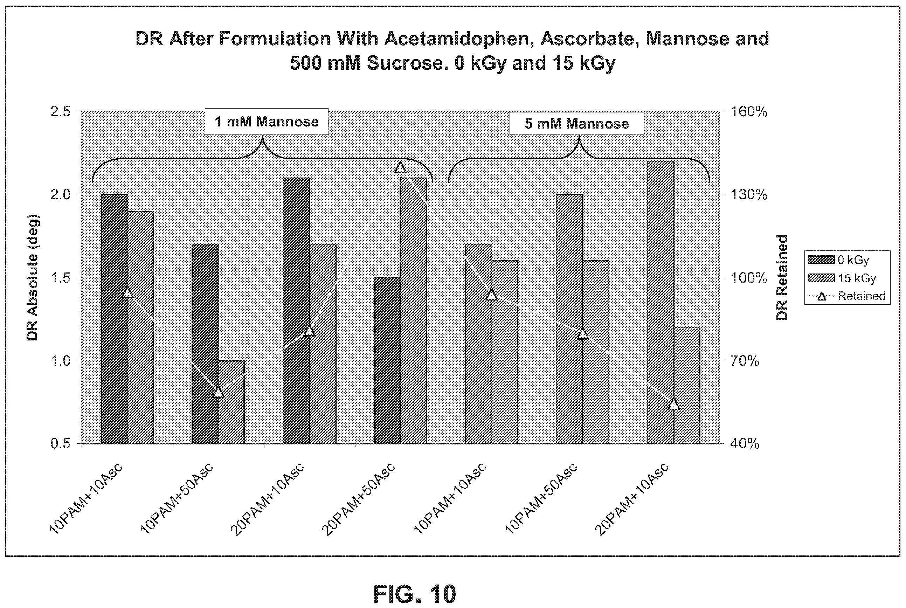

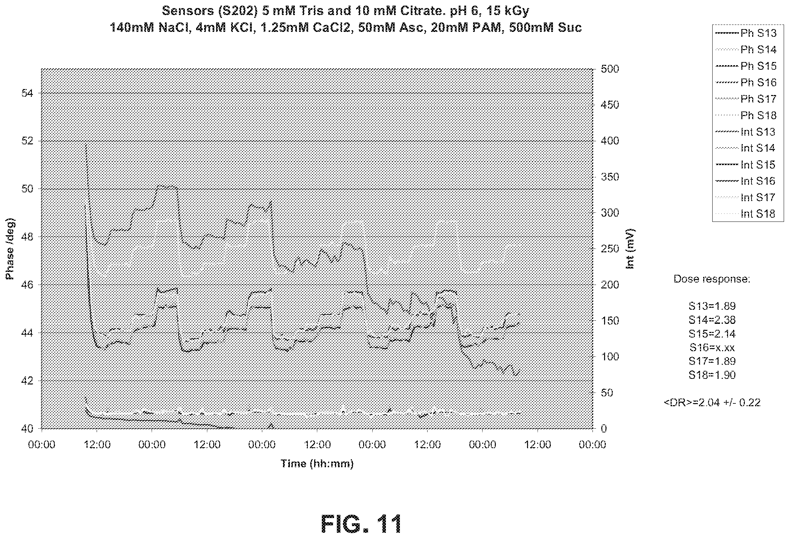

Figure 10 shows a bar graph of data presenting the absolute DR for both radiated and non-radiated sensor as a function of formulating the sensors with acetaminophen, ascorbic acid, mannose and 500 mM sucrose. An overall result is illustrated in Figure 11.

Figure 11 shows a graph of data showing sensor response after using Tris/Citrate saline buffer + excipients. Sensors show good retention of DR.

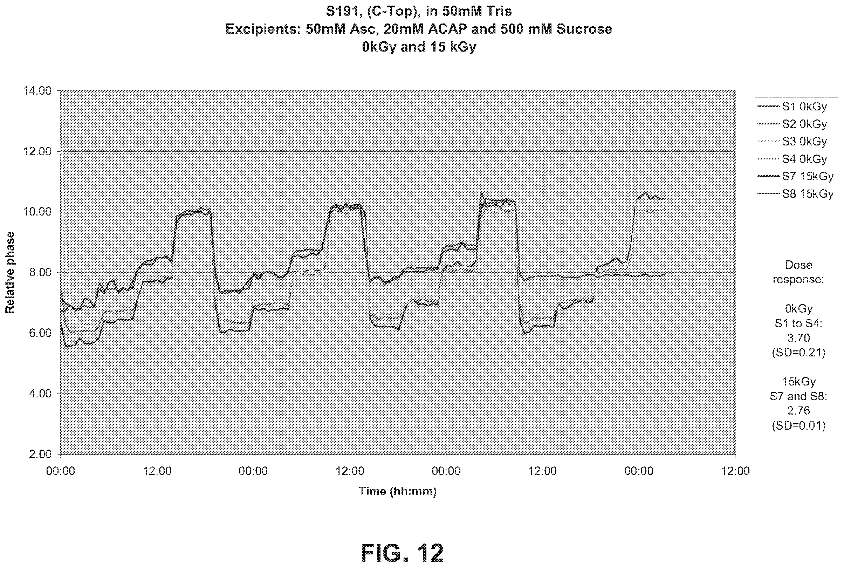

Figure 12 shows a graph of data presenting a direct comparison of e-beamed and non e-beamed sensors.

### Buffer impact on the sensor dose response retentions after e-beam

Due to a demand for not degrading the polymer used on the sensor pH level needs to be around 6 during wet storage.

### PBS Buffer results

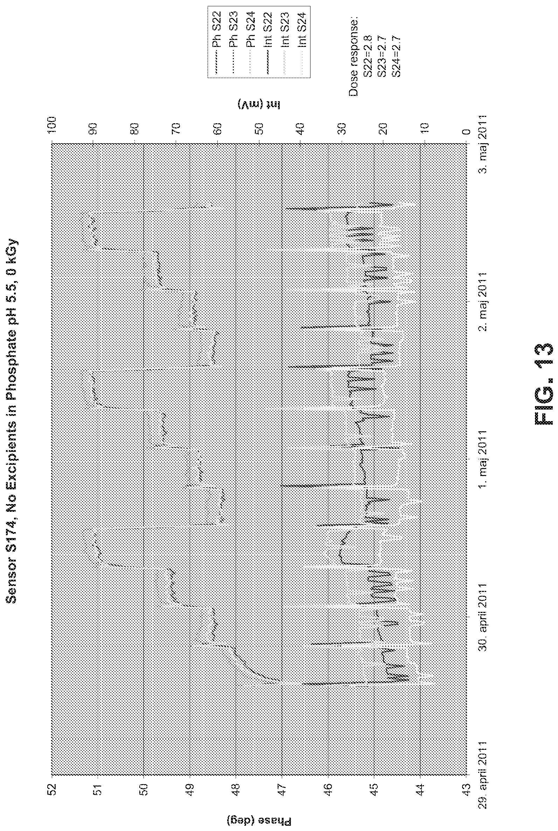

Figure 13 shows a graph of data obtained from a native sensor tested after storage in PBS pH=5.5. The sensor itself has no problem with the PBS buffer.

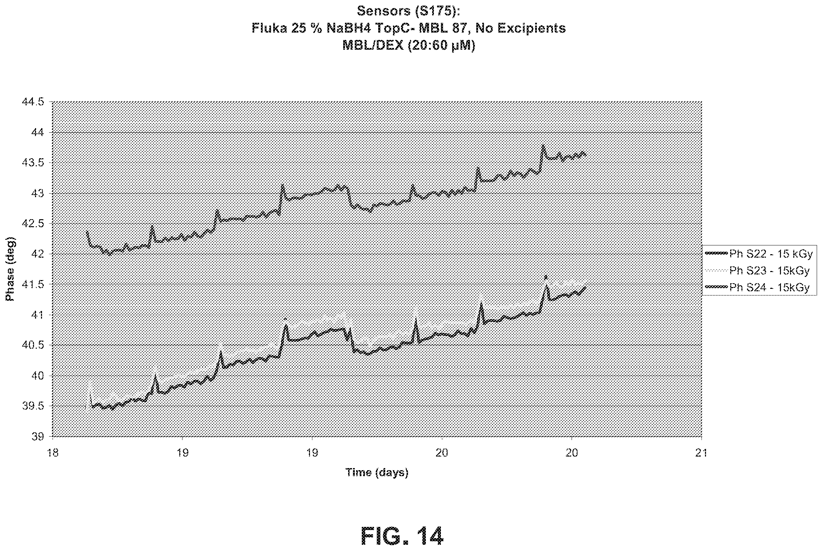

Figure 14 shows a graph of data obtained from a sensor with excipients added (500 mM sucrose, 20 mM acetaminophen and 50 mM ascorbate) in PBS buffer during e-beam.

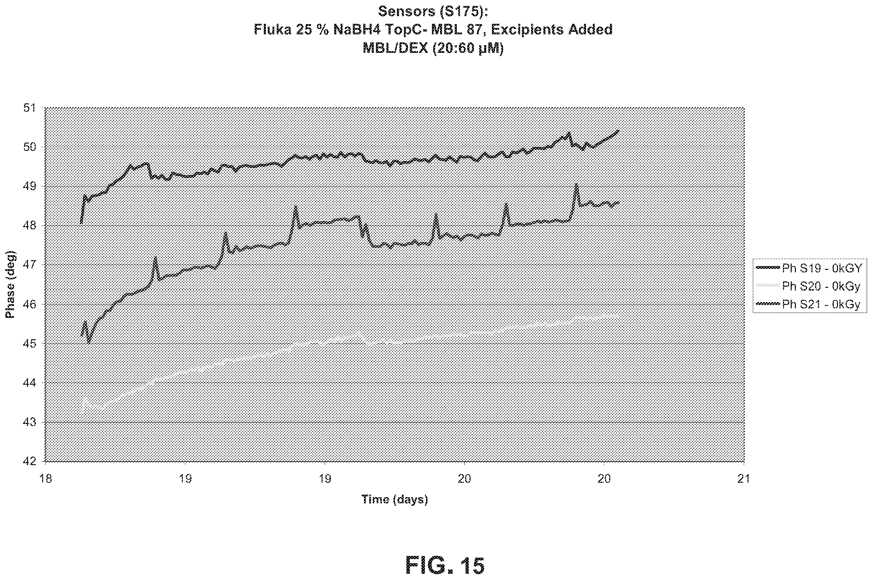

Figure 15 shows a graph of data obtained from a sensor with excipients added (500 mM sucrose, 20 mM acetaminophen and 50 mM ascorbate) in PBS buffer. No dose response and large drift is observed even though the sensors have not been e-beamed.

### Alternative buffers

Alternative clinically acceptable buffers are shown in Table 4.

**Table 4: List of optional buffers in the desired range together with their redox state.**

| **Buffer** | **pK1** | **pK2** | **pK3** | **Primary amine** | **"Red-Ox State"** | **Comment** |
|---|---|---|---|---|---|---|
| Phosphoric Acid | 2.15 | 7.20 | 12.33 | No | P=+7 | + Excipients DR Loss |
| Glycine | 2.35 | 9.78 | | Yes | C=+3 | |
| Alanine | 2.71 | 9.10 | | Yes | C=+3 | |
| **Tartaric Acid** | 3.04 | **4.37** | | No | C=+3 | |
| **Citrate** | 3.13 | **4.76** | 6.40 | No | C=+3 | |
| Lactate | 3.86 | | | No | C=+3 | |
| Ascorbic Acid | 4.17 | 11.57 | | No | C=+2 | |
| Acetic Acid | 4.76 | | | No | C=+3 | |
| Uric Acid | 5.83 | | | No | | Solubility problem |
| Carbonic acid/ Bicarbonate | 6.35 | 10.33 | | No | C=+4 | CO₂ pressure to keep pH |
| Tris | 8.06 | | | Yes | | |

Citrate was found to be superior, and tested in up to 50 mM concentration. Citrate works OK alone but better if Tris is added:

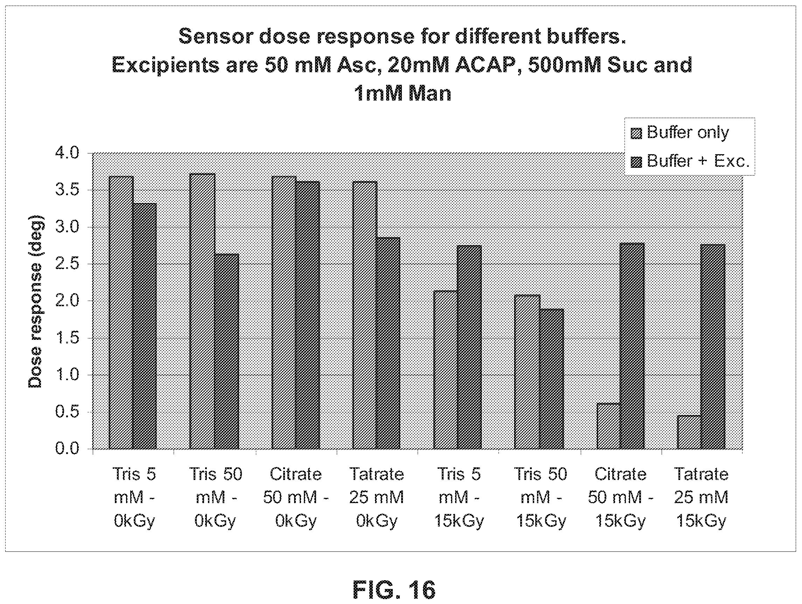

Figure 16 shows a bar graph of data on retained DR for using different buffer concentrations.

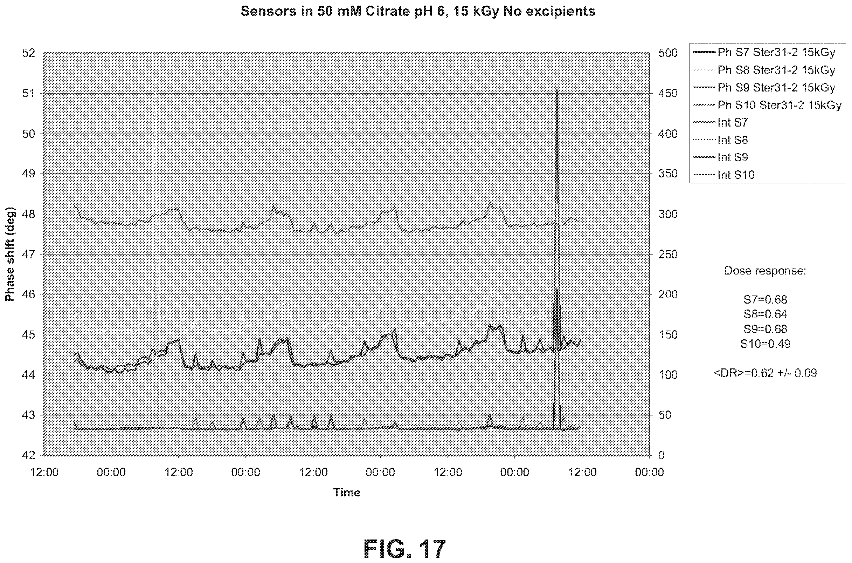

Figure 17 shows a graph of data resulting from sensors using citrate only during e-beam irradiation.

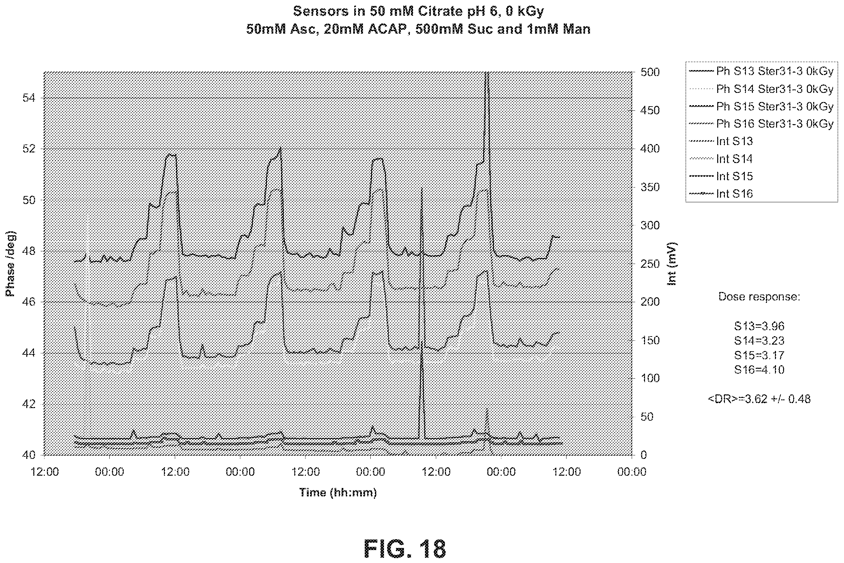

Figure 18 shows a graph of data resulting from sensors using citrate and excipients during e-beam irradiation.

### Amines to protect the chemistry from e-beam damages

In certain embodiments, amines can be included in the formulations (e.g. as a good quencher of radicals). Experimental results have shown that Tris (primary amine) by itself provides protection and that this protective effect is improved when excipients are added. Illustrative amines include urea, creatine, creatin, as well as the 20 naturally occurring amino acids.

The data in this Example confirms that the effects of a single excipient as well as the effects of combinations of excipients on glucose sensor DR retention following radiation sterilization are unpredictable. In these experiments, categories of agents tested included surfactants, amino acids (hydrophilic/hydrophobic), sugars (binding/non-binding), oxidants, antioxidants, drugs, bacteriostats, and combinations of these agents. The "best-in-class" excipients appear to include ascorbate, mannose, sucrose (high concentration) and acetaminophen (low concentration). The experimental data provides evidence that combinations of excipients can protect different specific sites or functionalities of a sensor against radiation damages. Ascorbate, mannose, sucrose and acetaminophen in combination provide particularly good signal retention for sensors. Typical embodiments of the invention include a combination of two to four excipients from each group and using a combination buffer consisting of 5mM Tris and/or 10 mM Citrate saline buffer. Some embodiments include sensor storage stability enhancing agents such as low-binding sugars (sucrose, trehalose and other polyols)

### Example 4: Sodium Nitrate and Other Reactive Oxygen Species (Ros) Excipients

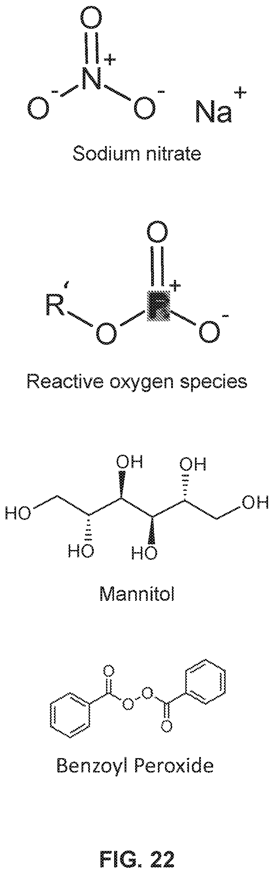

Illustrative experiments have demonstrated that the addition of sodium nitrate and/or other excipients improves the performance of sensor chemistry. Figure 22 shows the chemical structures of various protective excipients (i.e. sodium nitrate, reactive oxygen species, mannitol, and benzoyl peroxide) as described herein. Illustrative experiments have found that the sodium nitrate excipient is a more effective protectant than acetaminophen and ascorbate (see, e.g. Figure 19). Figure 20 is a graph illustrating the change of 50 mM sucrose, 320 mM mannitol, and 100 mM NaNO₃ formulation ratios over time. Further experiments have also demonstrated that the addition of sodium nitrate improves retention of dose response after e-beam, which is also an improvement over the protection offered by ascorbate and acetaminophen (see, e.g. Figure 21). The combination of sodium nitrate and mannitol increased retention to 90%.

Various protective excipients may be used. Sodium nitrate (NaNO₃) is an oxidizer and scavenger of free electrons caused by e-beam sterilization. It absorbs the electron and reduces into nitrate. Other nitrates with a similar functionality as sodium nitrate may also be used for e-beam protection.

Reactive oxygen species (ROS) are antioxidant compounds that may also be used to provide protection. These include ascorbate, glutathione, superoxide dismutase. In certain embodiments, the aqueous radioprotectant formulation comprises glutathione in a concentration range from 1 mM to 100 mM. Hydroxyethyl acrylate may also be used as a protectant because it can scavenge free radical electrons. In certain embodiments, the aqueous radioprotectant formulation comprises hydroxyethyl acrylate in a concentration range from 1 mM to 50 mM. Other monomers that undergo polymerization, such as PEG may also be used.

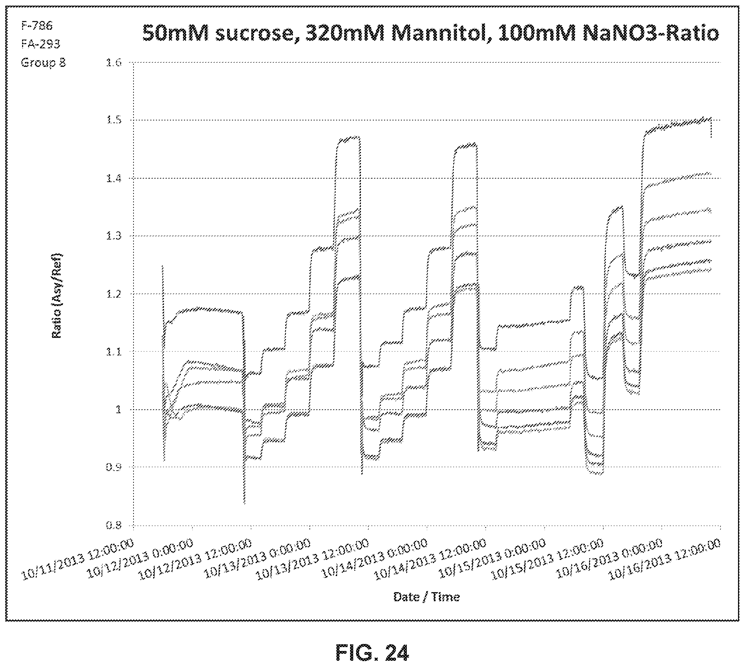

Mannitol is a sugar alcohol that is commonly used as a bulking agent, providing structure to freeze dried products. Other bulking agents could also be used to add freeze dried structure to the sensor chemistry. Illustrative experiments have demonstrated that replacing bicarbonate with mannitol in formulations increased sensor dose response by. about 8% (see Figure 23). Figure 24 shows a graph of the change of 50 mM sucrose, 320 mM mannitol, and 100 mM NaNO₃ formulation ratios over time.

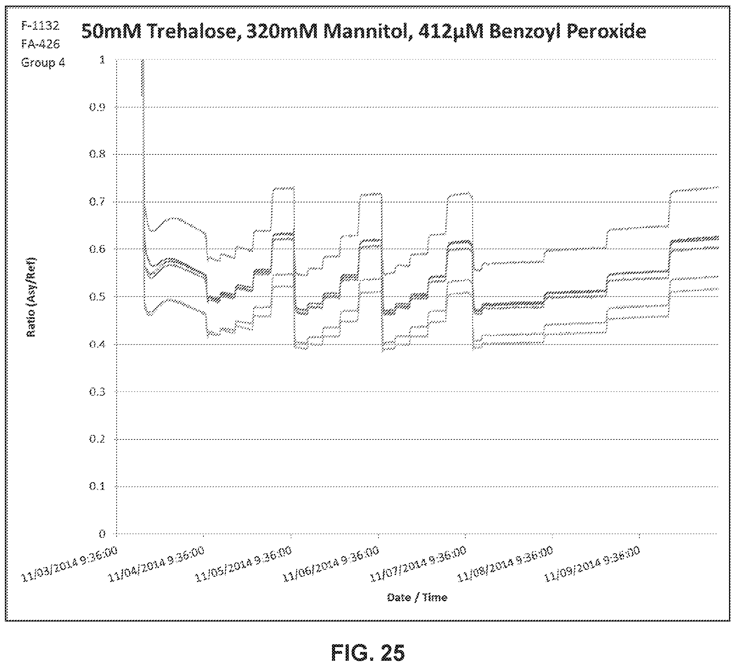

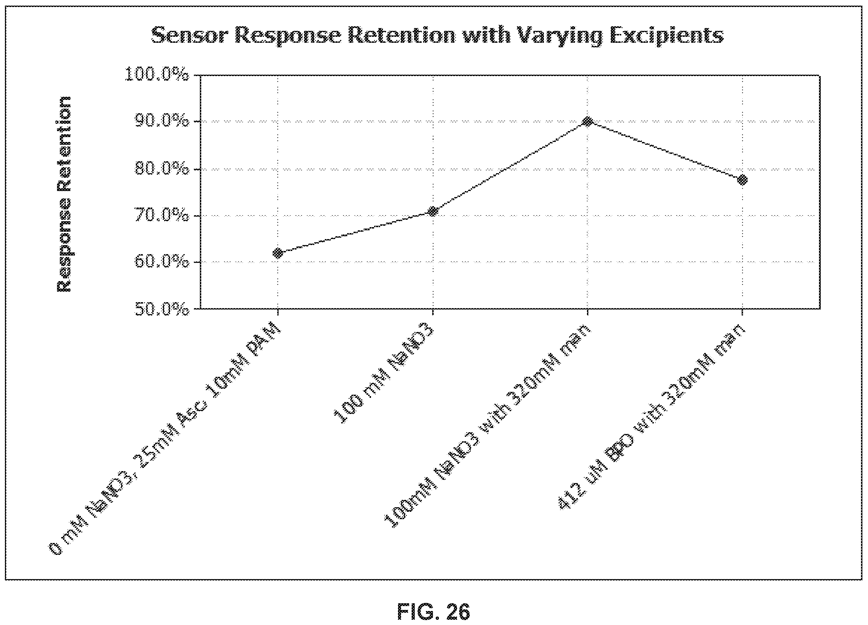

Benzoyl peroxide is an oxidizer and reactive oxygen species, acting as a protectant to e-beam radiation. Other moieties with radical electron forming capabilities are generally reactive oxygen species which will also act as e-beam protecting agents. Illustrative experiments have examined concentration of up to 500 µM for benzoyl peroxide as a protectant (see, e.g. Figure 26). Figure 25 shows a graph of the change of 50 mM trehalose, 320 mM mannitol, and 100 µM benzoyl peroxide formulation ratios over time.

### CONCLUSION

This concludes the description of the preferred embodiment of the present invention. The foregoing description of one or more embodiments of the invention has been presented for the purposes of illustration and description. It is not intended to be exhaustive or to limit the invention to the precise form disclosed. Many modifications and variations are possible in light of the above teaching.

## Claims

1. A method of inhibiting damage to a saccharide sensor that can result from a radiation sterilization process, the method comprising combining the saccharide sensor with an aqueous radioprotectant formulation during the sterilization process, wherein:
the saccharide sensor comprises a saccharide binding polypeptide having a carbohydrate recognition domain;
the aqueous radioprotectant formulation comprises a saccharide selected for its ability to bind the saccharide binding polypeptide, the aqueous radioprotectant formulation further comprises a reactive oxygen species that reactive oxygen species being sodium nitrate; and
performing the sterilization process under conditions selected so that the saccharide binds the saccharide binding polypeptide and the reactive oxygen species absorbs free electron energy generated by the radiation sterilization process, thereby inhibiting damage to the saccharide sensor.

2. The method of claim 1, wherein the radioprotectant formulation further comprises mannitol.

3. The method of claim 1, wherein the aqueous radioprotectant formulation comprises sodium nitrate, sucrose, and mannitol.

4. The method of claim 1, wherein the saccharide binding polypeptide comprises mannan binding lectin, concanavalin A or glucose oxidase.

5. The method of claim 1, wherein the saccharide comprises glucose, mannose, fructose, melizitose, N-acetyl-D-glucosamine, sucrose or trehalose.

6. The method of claim 1, wherein:
the saccharide sensor comprises a fluorophore; and
the aqueous radioprotectant formulation comprises a fluorophore quenching composition selected for its ability to quench the fluorophore.

7. The method of claim 1, wherein the radiation sterilization process comprises electron beam irradiation.

8. The composition of claim 1, wherein the aqueous radioprotectant formulation comprises sodium nitrate in a concentration of at least 100 mM to 1 M.

9. The composition of claim 2, wherein the aqueous radioprotectant formulation comprises mannitol in a concentration of at least 1 mM to 400 mM.

## Patentansprüche

1. Verfahren zum Verhindern von Beschädigung an einem Saccharidsensor, die aus einem Strahlensterilisationsverfahren resultieren kann, wobei das Verfahren das Kombinieren des Saccharidsensors mit einer wässrigen radioprotektiven Formulierung während des Sterilisationsverfahrens umfasst, wobei:
der Saccharidsensor ein Saccharidbindungspolypeptid mit einer Kohlenhydraterkennungsdomäne umfasst;
die wässrige radioprotektive Formulierung ein Saccharid umfasst, das aufgrund seiner Fähigkeit zum Binden des Saccharidbindungspolypeptids ausgewählt wird, wobei die wässrige radioprotektive Formulierung ferner eine reaktive Sauerstoffspezies umfasst, wobei die reaktiv Sauerstoffspezies Natriumnitrat ist; und
Durchführen des Sterilisationsverfahrens unter Bedingungen, die so ausgewählt sind, dass das Saccharid das Saccharidbindungspolypeptid bindet und die reaktive Sauerstoffspezies freie Elektronenenergie absorbiert, die durch das Strahlensterilisationsverfahren erzeugt wird, wodurch Beschädigung des Saccharidsensors verhindert wird.

2. Verfahren nach Anspruch 1, wobei die radioprotektive Formulierung ferner Mannit umfasst.

3. Verfahren nach Anspruch 1, wobei die wässrige radioprotektive Formulierung Natriumnitrat, Saccharose und Mannit umfasst.

4. Verfahren nach Anspruch 1, wobei das Saccharidbindungspolypeptid Mannan bindendes Lectin, Concanavalin A oder Glucoseoxidase umfasst.

5. Verfahren nach Anspruch 1, wobei das Saccharid Glucose, Mannose, Fructose, Melizitose, N-Acetyl-D-glucosamin, Saccharose oder Trehalose umfasst.

6. Verfahren nach Anspruch 1, wobei:
der Saccharidsensor ein Fluorophor umfasst; und
die wässrige radioprotektive Formulierung eine Fluorophor-Quenchzusammensetzung umfasst, die aufgrund ihrer Fähigkeit zum Quenchen des Fluorophors ausgewählt ist.

7. Verfahren nach Anspruch 1, wobei das Strahlensterilisationsverfahren Elektronenstrahl-Bestrahlung umfasst.

8. Zusammensetzung nach Anspruch 1, wobei die wässrige radioprotektive Formulierung Natriumnitrat in einer Konzentration von mindestens 100 mM bis 1 M umfasst.

9. Zusammensetzung nach Anspruch 2, wobei die wässrige radioprotektive Formulierung Mannit in einer Konzentration von mindestens 1 mM bis 400 mM umfasst.

## Revendications

1. Procédé d'inhibition d'un dommage à un capteur de saccharide, lequel peut résulter d'un processus de stérilisation par rayonnement, le procédé comprenant la combinaison du capteur de saccharide avec une formulation radioprotectrice aqueuse pendant le processus de stérilisation, dans lequel :
le capteur de saccharide comprend un polypeptide de liaison de saccharide ayant un domaine de reconnaissance de glucide ;
la formulation radioprotectrice aqueuse comprend un saccharide choisi pour son aptitude à lier le polypeptide de liaison de saccharide, la formulation radioprotectrice aqueuse comprend en outre une espèce réactive d'oxygène, cette espèce réactive d'oxygène étant du nitrate de sodium ; et
l'exécution du processus de stérilisation dans des conditions choisies de sorte que le saccharide lie le polypeptide de liaison de saccharide et l'espèce réactive d'oxygène absorbe l'énergie d'électrons libres générée par le processus de stérilisation par rayonnement, en inhibant de ce fait un dommage au capteur de saccharide.

2. Procédé selon la revendication 1, dans lequel la formulation radioprotectrice comprend en outre du mannitol.

3. Procédé selon la revendication 1, dans lequel la formulation radioprotectrice aqueuse comprend du nitrate de sodium, du saccharose et du mannitol.

4. Procédé selon la revendication 1, dans lequel le polypeptide de liaison de saccharide comprend de la lectine mannose-spécifique, de la concanavaline A ou de la glucose-oxydase.

5. Procédé selon la revendication 1, dans lequel le saccharide comprend du glucose, du mannose, du fructose, du mélézitose, de la N-acétyl-D-glucosamine, du saccharose ou du tréhalose.

6. Procédé selon la revendication 1, dans lequel :
le capteur de saccharide comprend un fluorophore ; et
la formulation radioprotectrice aqueuse comprend une composition de désactivation de fluorophore choisie pour son aptitude à désactiver le fluorophore.

7. Procédé selon la revendication 1, dans lequel le processus de stérilisation par rayonnement comprend une irradiation par faisceau d'électrons.

8. Composition selon la revendication 1, dans laquelle la formulation radioprotectrice aqueuse comprend du nitrate de sodium en une concentration d'au moins 100 mM à 1 M.

9. Composition selon la revendication 2, dans laquelle la formulation radioprotectrice aqueuse comprend du mannitol en une concentration d'au moins 1 mM à 400 mM.
